# EUROPÄISCHE PATENTSCHRIFT

(11) **EP 1 340 507 B1**
(45) Veröffentlichungstag und Bekanntmachung des Hinweises auf die Patenterteilung: **22.09.2004**
(21) Anmeldenummer: 02004299.0
(22) Anmeldetag: 28.02.2002
(51) Int. Cl.: A61K 38/17, A61K 48/00, G01N 33/50, G01N 33/68, C12Q 1/68, A61P 17/02

(54) **Verwendung eines Fibroblastenwachstumsfaktor-Bindeproteins zur Behandlung und Diagnose von diabetischen Wundheilugnsstörungen**
Use of fibroblast growth factor-binding protein for the treatment and diagnosis of diabetic wound healing
Utilisation de la protéine liant au facteur de croissance de fibroblastes pour le traitement et le diagnostic de la cicatrisation diabétique

(43) Veröffentlichungstag der Anmeldung: 03.09.2003
(73) Patentinhaber: Switch Biotech Aktiengesellschaft, 82061 Neuried (DE)
(72) Erfinder: Goppelt, Andreas, 80636 München (DE); Bittner, Michaela, 82319 Starnberg (DE)
(74) Vertreter: Bösl, Raphael, Dr. rer. nat., Dipl.-Chem.

(56) Entgegenhaltungen:
- WO-A-00/05416
- WO-A-00/47602
- US-A- 5 707 632
- US-B1- 6 255 454
- SAUTER EDWARD R ET AL: "Fibroblast growth factor-binding protein expression changes with disease progression in clinical and experimental human squamous epithelium." INTERNATIONAL JOURNAL OF CANCER, Bd. 92, Nr. 3, 2001, Seiten 374-381, XP002211897 ISSN: 0020-7136

### Sequenzprotokoll

<110> Switch Biotech Aktiengesellschaft
<120> Verwendung eines Fibroblastenwachstumsfaktor-Bindeproteins zur Behandlung und Diagnose von diabetischen Wundheilungsstörungen.
<130> S36968EP
<160> 12
<170> Windows NT
<210> 1
   <211> 234
   <212> PRT
   <213> Homo sapiens
   <223> humanes FGF-BP
<400> 1
<210> 2
   <211> 705
   <212> DNA
   <213> Homo sapiens
   <223> cDNA für humanes FGF-BP
<400> 2
   atgaagatct gtagcctcac cctgctctcc ttcctcctac tggctgctca ggtgctcctg  60
   gtggagggga aaaaaaaagt gaagaatgga cttcacagca aagtggtctc agaacaaaag 120
   gacactctgg gcaacaccca gattaagcag aaaagcaggc ccgggaacaa aggcaagttt 180
   gtcaccaaag accaagccaa ctgcagatgg gctgctactg agcaggagga gggcatctct 240
   ctcaaggttg agtgcactca attggaccat gaattttcct gtgtctttgc tggcaatcca 300
   acctcatgcc taaagctcaa ggatgagaga gtctattgga aacaagttgc ccggaatctg 360
   cgctcacaga aagacatctg tagatattcc aagacagctg tgaaaaccag agtgtgcaga 420
   aaggattttc cagaatccag tcttaagcta gtcagctcca ctctatttgg gaacacaaag 480
   cccaggaagg agaaaacaga gatgtccccc agggagcaca tcaagggcaa agagaccacc 540
   ccctctagcc tagcagtgac ccagaccatg gccaccaaag ctcccgagtg tgtggaggac 600
   ccagatatgg caaaccagag gaagactgcc ctggagttct gtggagagac ttggagctct 660
   ctctgcacat tcttcctcag catagtgcag gacacgtcat gctaa  705
<210> 3
   <211> 251
   <212> PRT
   <213> Mus musculus
   <223> murines FGF-BP
<400> 3
   Met Arg Leu His Ser Leu Ile Leu Leu Ser Phe 11
   Leu Leu Leu Ala Thr Gln Ala Phe Ser Glu Lys 22
   Val Arg Lys Arg Ala Lys Asn Ala Pro His Ser 33
   Thr Ala Glu Glu Gly Val Glu Gly Ser Ala Pro 44
   Ser Leu Gly Lys Ala Gln Asn Lys Gln Arg Ser 55
   Arg Thr Ser Lys Ser Leu Thr His Gly Lys Phe 66
   Val Thr Lys Asp Gln Ala Thr Cys Arg Trp Ala 77
   Val Thr Glu Glu Glu Gln Gly Ile Ser Leu Lys 88
   Val Gln Cys Thr Gln Ala Asp Gln Glu Phe Ser 99
   Cys Val Phe Ala Gly Asp Pro Thr Asp Cys Leu 110
   Lys His Asp Lys Asp Gln Ile Tyr Trp Lys Gln 121
   Val Ala Arg Thr Leu Arg Lys Gln Lys Asn Ile 132
   Cys Arg Asn Ala Lys Ser Val Leu Lys Thr Arg 143
   Val Cys Arg Lys Arg Phe Pro Glu Ser Asn Leu 154
   Lys Leu Val Asn Pro Asn Ala Arg Gly Asn Thr 165
   Lys Pro Arg Lys Glu Lys Ala Glu Val Ser Ala 176
   Arg Glu His Asn Lys Val Gln Glu Ala Val Ser 187
   Thr Glu Pro Asn Arg Val Lys Glu Asp Ile Thr 198
   Leu Asn Pro Ala Ala Thr Gln Thr Met Ala Ile 209
   Arg Asp Pro Glu Cys Leu Glu Asp Pro Asp Val 220
   Leu Asn Gln Arg Lys Thr Ala Leu Glu Phe Cys 231
   Gly Glu Ser Trp Ser Ser Ile Cys Thr Phe Phe 242
   Leu Asn Met Leu Gln Ala Thr Ser Cys 251
<210> 4
   <211> 756
   <212> DNA
   <213> Mus musculus
   <223> cDNA für murines FGF-BP
<400> 4
   atgagactcc acagcctcat cctgctctcc ttccttctcc tggctactca ggcgttctca 60
   gaaaaggtca gaaagagagc caagaacgca ccacacagca cagcggagga gggggtagag 120
   ggttcagctc cctcgttagg gaaggcccag aataagcaga gaagcaggac atctaaatct 180
   ctgacgcatg gcaagtttgt caccaaagac caagccacat gcagatgggc tgtgactgag 240
   gaggagcagg gcatcagcct gaaggtccag tgcacacaag ccgatcagga gttttcttgt 300
   gtttttgctg gtgacccaac tgactgcctt aaacacgaca aagaccagat ctactggaaa 360
   caggttgccc gcacgctgcg caaacagaaa aatatctgca ggaacgccaa gagtgtcttg 420
   aagaccagag tgtgcagaaa gagatttcca gagtctaacc tcaagctggt gaaccccaac 480
   gcacgtggaa acacgaagcc caggaaggag aaagcagagg tctccgcaag ggagcacaac 540
   aaggtccaag aagctgtctc cacggagcca aacagggtca aagaagacat cacactcaat 600
   ccagctgcga cccagaccat ggccattaga gatccagagt gtctagagga tccagatgtg 660
   ctcaaccaga ggaagaccgc cctggagttc tgtggggaat cttggagctc catttgcaca 720
   ttcttcctca acatgttaca ggcgacatca tgctaa 756
<210> 5
   <211> 23
   <212> DNA
   <213> künstliche Sequenz
<220>
   <223> Vorwärts-Primer für humanes FGF-BP
<400> 5
   gagctctctc tgcacattct tcc 23
<210> 6
   <211> 22
   <212> DNA
   <213> künstliche Sequenz
<220>
   <223> Rückwärts-Primer für humanes FGF-BP
<400> 6
   tagcatgacg tgtcctgcac ta 22
<210> 7
   <211> 20
   <212> DNA
   <213> künstliche Sequenz
<220>
   <223> Vorwärts-Primer für humanes Cyclophilin
<400> 7
   ggaatggcaa gaccagcaag 20
<210> 8
   <211> 20
   <212> DNA
   <213> künstliche Sequenz
<220>
   <223> Rückwärts-Primer für humanes Cyclophilin
<400> 8
   ggatactgcg agcaaatggg 20
<210> 9
   <211> 20
   <212> DNA
   <213> künstliche Sequenz
<220>
   <223> Vorwärts-Primer für murines FGF-BP
<400> 9
   cgttagggaa ggcccagaat 20
<210> 10
   <211> 22
   <212> DNA
   <213> künstliche Sequenz
<220>
   <223> Rückwärts-Primer für murines FGF-BP
<400> 10
   ttgccatgcg tcagagattt ag 22
<210> 11
   <211> 19
   <212> DNA
   <213> künstliche Sequenz
<220>
   <223> Vorwärts-Primer für murine GAPDH
<400> 11
   atcaacggga agcccatca 19
<210> 12
   <211> 20
   <212> DNA
   <213> künstliche Sequenz
<220>
   <223> Rückwärts-Primer für murine GAPDH
<400> 12
   gacatactca gcaccggcct  20

## Beschreibung

Die vorliegende Erfindung bezieht sich auf die Verwendung von Fibroblastenwachstumsfaktor-Bindeprotein (FGF-BP) Polypeptiden oder diese kodierenden Nukleinsäuren und/oder einer ein FGF-BP Polypeptid exprimierenden Zelle zur Behandlung, Diagnose und/oder Prävention von diabetes-assoziierten Wunden, sowie auf die Verwendung mindestens eines FGF-BP Polypeptids gemäß SEQ ID Nr. 1 und SEQ ID Nr. 3 und/oder mindestens einer für FGF-BP kodierenden Nukleinsäure gemäß SEQ ID Nr. 2 und SEQ ID Nr. 4 und/oder von gegen ein erfindungsgemäß verwendbares FGF-BP Polypeptid gerichteten Antikörpern oder Antikörperfragmenten und/oder einer ein FGF-BP Polypeptid exprimierenden Zelle zur Identifizierung von pharmakologisch aktiven Substanzen, die die Aktivität oder Expression von FGF-BP steigern.

Hautwunden gesunder Patienten heilen normalerweise ohne Probleme. Es sind jedoch eine Vielzahl zeitlicher und räümlicher Veränderungen der Zellzusammensetzung der Haut nötig, um vollständige Heilung des Gewebes zu erreichen. Dieser Prozess kann bis zu zwei Jahre andauern und ist bei nicht-foetalem Gewebe immer mit Narbenbildung verbunden. Dies weist auf die enorme Komplexität des Wundheilungsprozesses der Haut hin. Bei der Wundheilung können die zeitlich aufeinanderfolgenden, partiell überlappenden Phasen der Koagulation, Entzünwound healing, 1998, Oxford Institute for continuing education). Während der Koagulation aggregieren Blutplättchen, die Wachstums- und Koagulationsfaktoren freisetzen. Es wird eine Fibrin-Matrix gebildet, die die Zellmigration in der Wunde ermöglicht. Anschließend kommt es ca. 5-7 Tage nach der Verletzung zu einer Entzündungsreaktion. Dabei wandern verschiedene Zelltypen, vor allem neutrophile Granulozyten und Monozyten in die Wunde ein und setzen Mediatoren der Entzündungsreaktion frei. Die Proliferationsphase dient der Neubildung des verletzten Gewebes und der Restrukturierung des neugebildeten Gewebes. Die dabei beteiligten Prozesse umfassen vor allem die Neovaskularisierung, Fibroblastenproliferation und Reepithelialisierung durch Proliferation und Differenzierung verschiedenster Hautzellen und werden von verschiedensten Wachstumsfakoren und/oder Kombinationen davon gesteuert. Beispielsweise wird die Vaskularisierung durch die Wachstumsfaktoren bFGF, VEGF und TGF-beta 1 und 2 unterstützt, die von zerstörten Endothelzellen bzw. Makrophagen ausgeschüttet werden. Verschiedenste Zelltypen sind an der Ausschüttung von Faktoren beteiligt, die die Proliferation von Keratinozyten steuern, wie zum Beispiel EGF, TGF-alpha, bFGF, aFGF und KGF. Die Fibroblasten wiederum sezernieren mehrere Wachstumsfaktoren, z.B. PDGF und TGF-beta, die die Synthese und Einlagerung von Komponenten der extrazellulären Matrix (ECM) wie Fibronektin, Laminin, Glykosaminoglykane und Kollagen regulieren. Während der Reorganisation des Gewebes kommt es zu einer Umordnung der ECM-Komponenten, insbesondere des Kollagens. Durch den ständigen Abbau von Kollagen und dessen Neusynthese kann die reepithelialisierte Wunde reifen und es kommt innerhalb von zwei Jahren zur Bildung einer flachen Narbe. Wiederum sind eine Vielzahl von Wachstumsfaktoren und Chemoattraktanten für den koordinierten Umbau des Gewebes notwendig. So beeinflussen Interleukin 1, TNF-beta und Interferon-gamma die Sezernierung der ECM-Komponenten. Weiterhin sind TGF-beta, PDGF, VEGF und FGF essentiell für die Reorganisation.

Dieses exakt abgestimmte, komplexe Zusammenspiel der bei der Wundheilung auftretenden physiologischen Prozesse verdeutlicht, dass verschiedenste Störungen zu Beeinträchtigungen des Wundheilungsprozesses führen können. Einige dieser Faktoren sind beispielsweise das Alter, Immunerkrankungen, Ernährungsmängel, Zinkmangel, Innervationsstörungen, Alkoholmissbrauch oder genetische Defekte. Starke Beeinträchtigungen des Wundheilungsprozesses können dann zu chronischen Wunden und schließlich zu Ulzera führen. Bislang sind jedoch nur wenig zufriedenstellende Therapien entwickelt worden, um bei chronischen Wundheilungsstörungen eingreifen zu können. Etablierte Therapieformen beschränken sich auf physikalische Unterstützung der Wundheilung (z.B. Verbände, Kompressen, Gele), Ausschabung nekrotischen Gewebes und Transplantation von Hautgeweben, gezüchteten Hautzellen und/oder Matrixproteinen.

Da die Wachstumsfaktoren, wie oben aufgeführt, eine entscheidende Rolle für eine erfolgreiche Wundheilung und ein ästhetisches Ergebnis zu spielen scheinen, stellten in der Vergangenheit die Wachstumsfaktoren ein interessantes Objekt für die Therapieentwicklung dar. Da jedoch das korrekte Zusammenspiel der zahlreichen Wachstumsfaktoren von einer Vielzahl von Faktoren wie Menge, räumliche und zeitliche Verteilung sowie Kombination von Wachstumsfaktoren beeinflusst wird, gestaltet sich dieser Ansatz als äußerst kompliziert. So wurden in den letzten Jahren Wachstumsfaktoren zur Verbesserung der Wundheilung erprobt, bisher jedoch ohne die konventionelle Therapie entscheidend zu verbessern. Lediglich PDGF-BB ist zur Behandlung diabetischer Fußulzera zugelassen.

Ein bislang unbeachteter Aspekt bei Wundheilungsstörungen sind jedoch die pathogenen Hintergründe, die den jeweiligen Störungen zu Grunde liegen und aufgrund verschiedener molekularer Ursachen eine unterschiedliche Therapie erfordern. So werden üblicherweise venöse Ulzera molekular untersucht und stellvertretend für chronische und/oder schlecht heilende Wunden betrachtet. Unter chronischen Hautwunden werden jedoch sehr unterschiedliche Erkrankungen mit unterschiedlichem pathogenem Hintergrund zusammengefasst. Im allgemeinen werden als häufigste Vertreter chronischer Hautwunden diabetische Ulzera, venöse Ulzera, neuropathische Ulzera, arterielle Ulzera und Dekubitus Ulzera unterschieden. Dekubitus Ulzera sind durch längerfristige Druckeinwirkung bedingte, sehr tiefe Wunden, die mit Nekrose, Infektion und Mazeration des Gewebes einhergehen. Dagegen sind venöse Ulzera, die durch venöse Stasis hervorgerufen werden eher oberflächlich. Arterielle Ulzera werden dagegen häufig durch arterielle Verschlusskrankheiten verursacht. Diabetische Ulzera wiederum sind Ulzera die häufig bei Diabetes-Patienten vorkommen. Die Spät-Komplikationen der Diabetes umfassen neben einer Vielzahl von Erkrankungen auch charakteristische Hautveränderungen wie häufige Infekte, trophische Störungen und *Necrobiosis lipoidica.* Diese Veränderungen können sich zu schlecht heilenden Ulzera entwickeln. So leiden z.B. 25% der Patienten mit Typ-II-Diabetes häufig an chronischen Ulzera (z.B. "diabetischer Fuß"), von denen etwa die Hälfte aufwändige stationäre Behandlungen erfordern und letztlich doch schlecht heilen. Allein der diabetische Fuß verursacht mehr Klinikaufenthalte als jede andere mit Diabetes assoziierte Komplikation. Die Zahl dieser Fälle bei Diabetes Typ I und II ist im Steigen begriffen und repräsentiert ca. 2,5% aller Klinikeinweisungen.

Aufgabe der vorliegenden Erfindung ist es daher, einen neuen Wirkstoff zu finden, der insbesondere die Heilung, Diagnose und/oder Prävention von diabetes-assoziierten schlecht heilendenden Wunden entscheidend verbessert.

Überraschenderweise konnte nun gezeigt werden, dass diabetische Patienten im Gegensatz zu anderen Ulzera-Patienten selektiv zu einer verminderten Menge an FGF-BP neigen. Die in Beispiel 1 dargestellte Expressionsstudie der normalen Wundheilung zeigt, dass für eine normale Wundheilung eine erhöhte Expression von FGF-BP notwendig ist. Dies wird auch deutlich im Vergleich mit venösen Ulzera, wo eine permanente leichte Erhöhung der Expression von FGF-BP beoachtet wird. Hingegen besteht selektiv bei diabetischen Patienten eine Prädisposition für eine schlechtere Wundheilung, die durch eine verminderte Expression von FGF-BP gekennzeichnet ist. Dadurch lassen sich diabetische Ulzera gegenüber normalen Wunden und anderen Ulzera relativ gut abgrenzen.

Diese Ergebnisse, die in den Beispielen genau erläutert werden, werden im Folgenden zusammengefasst:

Biopsien aus humanen diabetischen Ulzera wiesen die für FGF-BP kodierende mRNA in deutlich geringerer Konzentration auf als Biopsien venöser Ulzera oder Biopsien normal heilender Wunden. Dies belegt, dass sich insbesondere diabetes-assoziierte Wundheilungsstörungen durch eine für diese Erkrankungen spezifisch verminderte Menge der Expression des FGF-BP auszeichnen. Durch die Identifizierung einer Patientengruppe, die unerwartet einen Mangel an FGF-BP aufweist, besteht die Möglichkeit, FGF-BP, das Protein kodierende Nukleinsäure(n), gegen ein erfinderisch verwendbares Polypeptid oder funktionell Varianten davon gerichtete Antikörper und/oder das Protein exprimierende Zellen für eine erfolgreiche und effektive Therapie und Diagnose diabetes-assoziierter Wunden einzusetzen. Diese Resultate konnten auch in Mäusen nachvollzogen werden, wodurch sich ein geeignetes Tiermodell für die Therapieentwicklung ergab. So zeigt ein Vergleich der Wundheilung zwischen normalen Mäusen und diabetischen Mäusen, die eine schlechtere Wundheilung aufwiesen, dass das FGF-BP in diabetischen Mäusen während der Wundheilung relativ zu intakter Haut im Durchschnitt um den Faktor 2,8 schwächer exprimiert ist.

Die Aufgabe wird daher erfindungsgemäß durch die Verwendung eines oder mehrer FGF-BP Polypeptide oder diese kodierende Nukleinsäuren gelöst.

Gegenstand der vorliegenden Erfindung ist daher die Verwendung mindestens eines FGF-BP Polypeptids gemäß der SEQ Nr. 1 und SEQ Nr. 3 oder der dafür kodierenden Nukleinsäure(n) gemäß der SEQ Nr. 2 und SEQ Nr. 4 oder gegen ein erfindungsgemäß verwendbares Polypeptid gerichteter Antikörper oder Antikörperfragmente oder einer FGF-BP exprimierenden Zelle zur Behandlung, Diagnose und/oder Prävention von diabetes-assoziierten Wunden. Eine bevorzugte diabetes-assoziierte schlecht heilende Wunde ist der diabetische Ulkus.

Ein weiterer Gegenstand der vorliegenden Erfindung ist die Verwendung mindestens eines FGF-BP Polypeptids gemäß SEQ ID Nr. 1 und 3 und/oder mindestens einer für FGF-BP kodierenden Nukleinsäure gemäß SEQ ID Nr. 2 und 4 oder gegen ein erfindungsgemäß verwendbares FGF-BP Polypeptid gerichteter Antikörper oder Antikörperfragmente und/oder mindestens einer FGF-BP Polypeptid exprimierenden Zelle zur in vitro Identifizierung pharmakologisch aktiver Substanzen, die die Funktion und/oder die Expression des FGF-BP Polypeptids und/oder einer für FGF-BP Polypeptid kodierenden Nukleinsäure steigern zur Behandlung und/oder Prävention von diabetes-assoziierten Wunden oder diabetischen Wundheilungsstörungen. Eine bevorzugte Verwendung der genannten Substanzen und Zellen ist dabei die Herstellung eines Tests zur Auffindung von pharmakologisch aktiven Substanzen in Zusammenhang mit diabetes-assoziierten Wunden.

Das humane FGF-BP ist ein Polypeptid mit einem Molekulargewicht von 17 kD welches als Monomer aber auch als Homodimer vorliegen kann. Es wurde ursprünglich aus Kulturmedium isoliert, welches durch humane epidermoide Karzinomzellen konditioniert worden war (Wu et al. (1991) J. Biol. Chem. 5:16778-16785). Weiterhin wurde eine starke Erhöhung der Expression von FGF-BP vor allem in frühen Stadien schwammartiger Karzinome, in normalen und immortalisierten humanen Keratinozyten, sowie in Nierentubuli bei renalen Erkrankungen, wie z.B. HIV-assoziierter Nephropathie oder hämolytischem urämischem Syndrom (HUS) beobachtet (Liu et al. (2001) Kidney Int., 59:1717-1728). Die Publikation Sauter et al. ((2001), Int. J. Cancer 92:374-381) beschreibt allerdings, dass FGF-BP bei der Bildung invasiver Karzinome keine wichtige Rolle spielt.

FGF-BP ist bekannt dafür, dass es FGF-1 (aFGF) und FGF-2 (bFGF) nichtkovalent und reversibel bindet (Wu et al. (1991) J. Biol. Chem. 5:16778-16785). FGF-1 und -2 gehören zur Familie der FGF Proteine, der über 20 verschiedene Proteine angehören und die in verschiedensten Geweben exprimiert werden. FGFs sind sogenannte translozierenden Proteine, die unabhängig vom normalen Sekretionsweg sezerniert werden und an die extrazelluläre Matrix binden, wodurch sie einerseits vor Degradation geschützt werden und andererseits ein lokales Reservoir an inaktivem Protein bilden, das eine strikte räumliche Regulation des FGF Signalweges erlaubt. Die Freisetzung von FGF aus der extrazellulären Matrix ermöglicht die Bindung von FGF an seinen Rezeptor. So wird beispielsweise bFGF von verschiedensten Zelltypen, wie Keratinozyten, Fibroblasten, Endothelzellen und Makrophagen sezerniert und übt seinerseits, entweder autokrin oder parakrin, chemotaktische und mitogene Einflüsse aus (Powers et al. (2000) Endocr. Relat. Cancer 7:165-197).

Gegenwärtig sind zwei Mechanismen für die Freisetzung der FGFs bekannt. Der erste Mechanismus beinhaltet die enzymatische Spaltung durch Plasmin oder Heparanase, der zweite die Bindung an FGF-BP als Carrierprotein. Die Anwesenheit von FGF-BP kann somit dazu beitragen, die Aktivität der FGFs zu erhöhen. Eine solche stimulierende Wirkung wurde bisher für Angiogenese und Proliferation exklusiv im Zusammenhang mit Tumorentstehung und -wachstum nachgewiesen. So konnte gezeigt werden, dass rekombinantes FGF-BP ohne Kofaktoren direkt an bFGF binden, gleichzeitig die Affinität des bFGF für Heparin reduzieren und die Proliferation von 3T3 Fibroblasten und Endothelzellen stimulieren kann. Somit ist das FGF-BP in hohem Maße an der Entstehung und Progression von Tumoren beteiligt und wurde ausschließlich in diesem Zusammenhang diskutiert. Dies wird zusätzlich durch den beobachteten synergistischer Effekt von bFGF und FGF-BP auf die Angiogenese im "Chorioallantois-Membrane-Assay" bestätigt. Dieser parakrine Effekt konnte mittels Antikörper gegen FGF2 vollständig blokkiert werden (Tassi et al. (2001) J. Biol. Chem. 276:40247-53; Aigner et al. (2001) Int. J. Cancer 92:510-517). Weiterhin führte die *in vitro* Ablation von FGF-BP mittels Ribozymen in Kolonkarzinomzellen zu einer verminderten Freisetzung von biologisch aktivem bFGF (FGF2) sowie zu reduziertem Tumorwachstum und Angiogenese in xenotransplantierten Tumoren (Czubayko et al. (1997) Nat. Med. 3:1083-1084; WO 99/15703). Die tumorfördernde Wirkung von FGF-BP spiegelte sich auch in der Regulation der Transkripte wieder. So wurden FGF-BP Transkripte von Faktoren, die die Differenzierung steuern, wie z.B. Retinsäure herunterreguliert (Liaudet-Coopman et al. (1996) J. Biol. Chem. 271:21303-21308). Hingegen wurden FGF-BP Transkripte von tumorwachstumsfördernden Faktoren, wie z.B. 12-O-Tetradecanoylphorbol-13-Acetat (TPA), Serum und dem sogenannten Epidermal Growth Factor (EGF) hochreguliert (siehe z.B. Harris et al. (1998) J. Biol. Chem. 273:19130-19139).

Untersuchungen an Mäusen ergaben bisher, dass FGF-BP die Funktion der FGFs während der Embryogenese in der Haut stimuliert und in adulter Haut offenbar herunterreguliert wird. Erst in frühen Stadien der Karzinogenese wird eine starke Reexpression von FGF-BP *in vivo* und *in vitro* in der Haut beobachtet (Kurtz et al. (1997) Oncogene 14:2671-2681). Interessanterweise wurde jedoch bisher kein Zusammenhang zwischen FGF-BP und der Freisetzung von FGFs aus der extrazellulären Matrix zur Vermittlung von Proliferation und Angiogenese während gestörter Wundheilungsprozesse nachgewiesen oder nahegelegt, obwohl eine wundheilungsfördernde Wirkung von aFGF und bFGF in verschiedensten Tiermodellen gezeigt wurde (Quirinia&Viidik et al. (1998) Scand. J. Plast. Reconstr. Surg. Hand. Surg. 32:9-18; Fu et al. (1998) Lancet 352 :1661-4; Okumura et al. (1996) Biol. Pharm. Bull. 19:530-5; Mellin et al. (1995) J. Invest. Dermatol. 104 :850-5). Im Gegenteil, FGFs, die in der Wundheilung eine Rolle spielen, werden gemäss dem gegenwärtigen Stand der Technik hauptsächlich unspezifisch durch mechanische Verletzung der Zellen freigesetzt (McNeil et al. (1989) J. Cell Biol. 109:811-22).

Die überraschende, selektiv in diabetes-assoziierten Wunden verringerte Menge an FGF-BP führt jedoch zu einer völlig neuen Ausgangssituation: Die schlechtere Wundheilung diabetischer Patienten steht in Zusammenhang mit der ungenügenden lokalen Freisetzung der vorhandenen Reserven an FGF. Somit trägt die vorliegende Erfindung zur Überwindung dieses technischen Vorurteils, die schlechtere Wundheilung bei diabetischen Patienten sei in der verminderten Verfügbarkeit von FGF begründet, bei und eröffnet die Möglichkeit, die Aktivität von FGFs bei Wundheilungsstörungen, insbesondere diabetes-assoziierte Wunden, durch lokale Gabe von FGF-BP entscheidend zu erhöhen.

Da eine erhöhte Expression von FGF-BP und die damit verbundene Regulation von FGF bislang experimentell nur mit der Entstehung und Progression von Tumorerkrankungen in Zusammenhang gebracht wurde, wurde die Gabe eines solchen Proteins und/oder Nukleinsäure zum Einsatz für die Behandlung von Krankheiten bisher gar nicht in Betracht gezogen. Die überraschende Identifikation von Wundheilungsstörungen, die durch eine verringerte Menge an FGF-BP gekennzeichnet sind, insbesondere von diabetes-assoziierten schlecht heilenden Wunden, bietet jedoch erstmals die Möglichkeit FGF-BP, dieses Protein kodierende Nukleinsäure(n), gegen FGF-BP gerichtete Antikörper und/oder Zellen, die dieses Protein exprimieren, zur Behandlung, Diagnose und/oder Prävention dieser Wundheilungsstörungen einzusetzen.

Die Wundheilung und deren krankhafte Störungen im Sinne der Erfindung sind abzugrenzen von Hauterkrankungen, die mit einer entarteten Zellentwicklung und Zelldifferenzierung einhergehen, insbesondere von Hautkrebs. Bei letzterer Erkrankung kommt es zur Transformation einzelner Zellen, die dadurch unkontrolliert, autonom, d.h. von Interaktionen mit anderen Zelltypen isoliert zu proliferieren beginnen und dabei die pathologischen Veränderungen an die Tochterzellen vererben. Es handelt sich also um eine Erkrankung, die mit einem Verlust von Interaktionen, beispielsweise von Zell-Zell Adhäsion und von typischen Zelleigenschaften einhergeht. Hingegen beruhen Wundheilungsstörungen im Sinne der Erfindung auf Störungen von Hautzellen in ihrem physiologischen Kontext. So kann der Verlauf der Wundheilung durch verschiedenste endogene und exogene Faktoren moduliert sein. Bereits kleine Störungen der Interaktionen zwischen den unterschiedlichen Zelltypen der Dermis und Epidermis selbst, aber auch Wechselwirkungen mit anderen Geweben und Organen wie dem Blutgefäßsystem, dem Nervensystem und dem Bindegewebe, können zu gestörter Wundheilung gefolgt von Narbenbildung führen. Weiterhin können Infektionen, Alterung, Erkrankungen, wie Diabetes und Immunerkrankungen sowie Vitaminmängel den Wundheilungsprozess beeinträchtigen. Beispiele für gestörte Wundheilung sind Wunden diabetischer Patienten und Alkoholiker, mit Mikroorganismen infizierte Wunden, ischämische Wunden und Wunden von Patienten mit mangelnder Durchblutung und Venenstau. Besonders bevorzugte schlecht heilende Wunden sind diabetische, neuropathische, venösen und arterielle Ulzera, insbesondere diabetes-assozierte Wunden.

Der autonome Charakter der Krebserkrankungen zeigt sich auch auf therapeutischer Ebene. Im Falle nicht-metastasierender Tumore läßt sich Krebs chirurgisch behandeln ist. Diese physikalische Behandlungsmöglichkeit ist möglich, da zwischen Tumorzellen und den umliegenden Zellen und Geweben keine Interaktionen stattfinden, so das durch einfache Exzision des Tumors der Patient geheilt werden kann, während dies bei Wundheilungsstörungen im Sinne der Erfindung nicht möglich ist - die krankhaften Störungen der Zell-Zell und/oder Gewebe-Gewebe Interaktionen können nicht durch Herausschneiden betroffener Hautstellen behoben werden. Dass es sich bei den verglichenen Krankheiten um Krankheiten handelt, denen ein grundsätzlich unterschiedlicher Mechanismus zugrunde liegt, wird deutlich, wenn man die therapeutischen Ansätze vergleicht. Bei Krebserkrankungen und Krankheiten, die mit einer entarteten Zellproliferation einhergehen, ist die Therapie auf die Abtötung schnell wachsender Zellen z.B. mittels Cytostatika gerichtet. Diese toxischen Substanzen verhindern das Wachstum aktiv proliferierender Zellen während Zellen in der G0-Phase des Zellzyklus nicht beeinflusst werden. Hingegen zielt die Behandlung von Wundheilungsstörungen im Sinne der Erfindung auf die Modulation der Interaktionen zwischen den verschiedenen Zelltypen, wie z.B. durch die Beeinflussung der Migration, Proliferation und Differenzierung einzelner Zelltypen. Wundheilungsstörungen im Sinne der Erfindung können nicht durch generelle Inaktivierung proliferierender Zellen geheilt werden. Der methodische Ansatz zur Identifizierung der erfindungsgemäß verwendeten Nukleinsäuren, die an der Wundheilung und deren Störungen im Sinne der Erfindung beteiligt sind unterscheidet sich deutlich von Verfahren, die geeignet sind Nukleinsäuren zu identifizieren, die an Prozessen der Krebserkrankungen beteiligt sind. Letztere können durch Analyse differentiell exprimierter Gene des vom Krebs betroffenen Zelltyps identifiziert werden. Ziel des Assays der vorliegenden Erfindung ist es jedoch vielmehr, durch Vergleich der Expression in kranken und gesunden Gewebebiopsien Gene zu identifizieren, die an den komplexen Prozesse der Wundheilung und deren krankhaften Störungen beteiligt sind. Zur Identifizierung von krebsrelevanten Gene wäre dieses Verfahren ungeeignet.

Unter "Wundheilung" im Sinne der vorliegenden Erfindung ist der Heilungsprozess einer mechanischen Verletzung der Haut zu verstehen, wie z.B. Schnittwunden, Schürfwunden oder das Aufreiben der Haut bespielsweise durch andauernde Belastung, beispielsweise Dekubitus oder nekrotische Prozesse, beispielsweise *Nekrobiosis lipoidica*.

Beispiele für "Wundheilungsstörungen" im Sinne der vorliegenden Erfindung sind Wunden diabetischer Patienten und Alkoholiker, mit Organismen oder Viren infizierte Wunden, ischämische Wunden, Wunden von Patienten mit arteriellen Verschlusskrankheiten oder venöser Insuffizienz, und Narben, bevorzugt überschießende Narben, insbesondere Keloide. Besonders bevorzugte schlecht heilende Wunden sind diabetische, neuropathische, venöse und arterielle Ulzera, insbesondere diabetes-assoziierte Wunden.

Unter "diabetes-assoziierten Wunden" im Sinne der vorliegenden Erfindung, sind Hautwunden von nicht-menschlichen Säugetieren und Menschen mit Diabetes mellitus zu verstehen. Beispiele für solche Hautwunden sind durch Diabetes verursachte Ulzera, beispielsweise *Ulcus cruris arteriosum* oder die *Necrobiosis lipoidica*.

Unter "Aktivität" im Sinne der vorliegenden Erfindung ist die Interaktion zwischen FGF-BP Polypeptiden selbst (Homodimere) aber auch zwischen FGF-BP Polypeptiden und verschiedenen anderen Molekülen, wie beispielsweise Perlecan (ein Heparansulfat-Proteoglycan), Heparin und FGF-Familienmitgliedern, wie z.B. FGF-1 oder FGF-2, sowie die dadurch hervorgerufenen physiologischen, funktionellen, Proliferations- und Differenzierungsveränderungen von Zellen und/oder Zellverbänden zu verstehen. Beispiele solcher Aktivitäten finden sich bei Mongiat et al. (2001), J. Biol. Chem. 276: 10263-10271 und Tassi et al. (2001) J. Biol. Chem. 276:40247-53.

Unter einer "verringerten Menge an FGF-BP" im Sinne vorliegender Erfindung, ist sowohl eine verringerte Expression an FGF-BP Polypeptiden und FGF-BP kodierende Nukleinsäuren als auch eine verringerte Aktivität von FGF-BP Polypeptiden zu verstehen.

Der Begriff "funktionelle Varianten" eines Polypeptids im Sinne der vorliegenden Erfindung umfasst Polypeptide, die beispielsweise wie die erfindungsgemäß verwendeten Polypeptide während Hauterkrankungen, die mit erniedrigten Spiegeln an FGF-BP einhergehen, reguliert werden.

"Funktionelle Varianten" im Sinne der vorliegenden Erfindung sind auch Polypeptide, die eine Sequenzhomologie, insbesondere eine Sequenzidentität, von ca. mindestens 70%, vorzugsweise ca. mindestens 80%, insbesondere ca. mindestens 90%, vor allem ca. mindestens 95% zu dem Polypeptid mit der Aminosäuresequenz gemäß einer der SEQ ID Nr. 1 und SEQ ID Nr. 3 aufweisen. Unter "Sequenzidentität" versteht man dabei die Übereinstimmung (= % Positive) zwischen zwei Sequenzen, die im Falle von Polypeptidsequenzen beispielsweise mittels BlastP 2.0.1 und im Falle von Polynukleotidsequenzen beispielsweise mit BLASTN 2.0.14 bestimmt wird, wobei der Filter=off gesetzt ist und BLOSUM 62 ist (Altschul et al. (1997) Nucleic Acids Res. 25:3389-3402).

Beispiele solcher funktionellen Varianten sind demnach die zu einem erfindungsgemäß verwendbaren Polypeptid homologen Polypeptide, die aus anderen Organismen als dem Menschen bzw. der Maus, vorzugsweise aus nicht-menschlichen Säugetieren wie z.B. Affen, Schweinen und Ratten stammen. Andere Beispiele von funktionellen Varianten sind Polypeptide, die durch unterschiedliche Allele des Gens in verschiedenen Individuen oder in verschiedenen Organen eines Organismus kodiert werden. Weitere Beispiele für funktionelle Varianten sind beispielsweise auch Polypeptide, die von einer FGF-BP kodierenden Nukleinsäure kodiert werden, die aus nicht-hautspezifischem Gewebe, z.B. Embryonalgewebe, isoliert werden, jedoch nach Expression in einer an der Wundheilung beteiligten Zelle die bezeichneten Funktionen besitzen.

Um festzustellen, ob es sich bei einem Polypeptid um eine funktionelle Variante eines erfindungsgemäß verwendbaren Polypeptids handelt, kann mittels funktionaler Tests die Aktivität des zu prüfenden Polypeptids mit der Aktivität eines erfindungsgemäß verwendbaren Polypeptids verglichen werden. Unter der Vorraussetzung, dass das zu prüfende Polypeptid die Anforderungen an eine funktionelle Variante auf der Ebene der Sequenzidentität erfüllt, handelt es sich bei dem zu prüfenden Polypeptid dann um eine funktionelle Variante, wenn dessen Aktivität im funktionalen Test mit der des erfindungsgemäß verwendbaren Polypeptids ähnlich oder identisch ausfällt.

Diese funktionalen Tests umfassen beispielsweise die Applikation eines Expressionsvektors, der eine das zu prüfendende Polypeptid kodierende Nukleinsäure enthält oder die Applikation des zu prüfenden Polypeptids selbst (Beispiel 4) oder eines gegen das zu prüfende Polypeptid gerichteten Antikörpers oder eines Antisense-Oligonukleotids auf Wunden. Nach Inkubation, beispielsweise eines Expressionsvektors, wird der Fortgang der Wundheilung bei Gabe der unterschiedlichen Expressionsvektoren, enthaltend entweder eine das zu prüfendende Polypeptid kodierende Nukleinsäure oder eine das erfindungsgemäß verwendbare Polypeptid kodierende Nukleinsäure oder einen Expressionsvektor ohne Insert verglichen. Diese funktionalen Tests können beispielsweise auch auf die Aktivität des zu prüfenden Polypeptids bei Störungen der Wundheilung, zum Beispiel bei schlecht heilenden, Dexamethason-behandelten oder diabetischen Wunden von Tieren, angewandt werden. So führte beispielsweise die Applikation von PDGF-A und PDGF-B Polypeptiden auf schlecht heilende Wunden von Kaninchen zu vergleichbarer Verbesserung der Wundheilung (Tyrone, JW et al. (2000) J. Surg. Res. 93:230-236).

Funktionelle Varianten des Polypeptids können auch Teile des erfindungsgemäß verwendeten Polypeptids mit mindestens 6 Aminosäuren Länge, vorzugsweise mit mindestens 8 Aminosäuren Länge, insbesondere mit mindestens 12 Aminosäuren Länge sein. Umfasst sind auch N- und/oder C-terminale und/oder interne Deletionen des erfindungsgemäß verwendeten Polypeptids im Bereich von ca. 1-60, vorzugsweise von ca. 1-30, insbesondere von ca. 1-15, vor allem von ca. 1-5 Aminosäuren. Beispielsweise kann die erste Aminosäure Methionin fehlen, ohne dass die Funktion des Polypeptids wesentlich verändert wird.

Die erfindungsgemäß verwendbaren Polypeptide können weiterhin dadurch gekennzeichnet sein, dass diese synthetisch hergestellt werden. So kann das gesamte Polypeptid oder Teile davon zum Beispiel mit Hilfe der klassischen Synthese (Merrifield-Technik) synthetisiert werden. Teile der erfindungsgemäßen Polypeptide eignen sich insbesondere zur Gewinnung von Antiseren, mit deren Hilfe geeignete Genexpressionsbanken durchsucht werden können, um so zu weiteren funktionellen Varianten des erfindungsgemäßen Polypeptids zu gelangen.

Erfindungsgemäß verwendbare Polypeptide können nach allgemein bekannten rekombinanten Verfahren hergestellt werden. Dabei wird das Polypeptid beispielsweise durch Expression der Nukleinsäuren nach SEQ ID Nr. 2 und 4 oder funktioneller Varianten davon in einem geeigneten Expressionssystem nach dem Fachmann allgemein bekannten Methoden hergestellt. Diese Expressionssysteme werden weiter unten erläutert. Als Zellen eignen sich beispielsweise die E. coli Stämme DHS, HB101 oder BL21, der Hefestamm *Saccharomyces cerevisiae*, die Insektenzelllinie Lepidopteran, z.B. von *Spodoptera frugiperda*, oder die tierischen Zellen COS, Vero, 293, HaCaT, und HeLa, die alle allgemein erhältlich sind. So konnte das erfindungsgemäße FGF-BP bereits erfolgreich in Sf-9 Insektenzellen exprimiert werden ((Beispiel 4) Tassi et al. (2001) J. Biol. Chem. 276:40247-53).

Weiterhin können erfindungsgemäß verwendbare Polypeptide aus einem Organismus oder aus Gewebe oder Zellen isoliert und erfindungsgemäß verwendet werden. So ist es beispielsweise möglich, erfindungsgemäß verwendbare Polypeptide aus Säugetiergewebe, beispielsweise aus Haut oder aus Körperflüssigkeiten, beispielsweise Blut, Serum, Speichel, Synovialflüssigkeit oder Wundflüssigkeit aufzureinigen. Weiterhin können aus Zellen, die erfindungsgemäß verwendbare Polypeptide exprimieren, Zelllinien hergestellt werden, die dann zur Isolierung von erfindungsgemäß verwendbaren Polypeptiden verwendet werden können. Beispielsweise können Expressionsvektoren, enthaltend die erfindungsgemäß verwendbaren Nukleinsäuren, in Hautzellen, beispielsweise HaCaT Zellen transformiert werden. Die Expression kann beispielsweise konstitutiv oder induzierbar sein. So konnte das erfindungsgemäße FGF-BP Protein bereits aus durch Zelllinien konditioniertem Medium bzw. aus bovinem Brustdrüsengeweben erfolgreich gereinigt werden (Wu et al. (1991) J. Biol. Chem. 266:16-778-85; Wang et al. (1998) Biochem. Mol. Biol. Int. 46:81-87; Lametsch et al. (2000) J. Biol. Chem. 275:19469-19474).

Eine weitere Ausführungsform betrifft die Verwendung der erfindungsgemäßen FGF-BP Polypeptide, wobei die Varianten von SEQ ID Nr. 1 und/oder 3 bzw. SEQ ID Nr 2 und/oder 4 Fusionsproteine bzw. für Fusionsproteine kodierende Nukleinsäuren sind. Erfindungsgemäß verwendbare Fusionsproteine können beispielsweise hergestellt werden, indem erfindungsgemäß verwendbare Nukleinsäuren in einer geeigneten Zelle exprimiert werden.

Die Fusionsproteine selbst weisen bereits die Funktion eines Polypeptids der Erfindung auf oder sind erst nach Abspaltung des Fusionsanteils funktionell. Vor allem zählen hierzu Fusionsproteine mit einem Anteil von ca. 1-300, vorzugsweise ca. 1-200, insbesondere ca. 1-100, vor allem ca. 1-50 fremden Aminosäuren. Beispiele solcher Peptidsequenzen sind prokaryotische Peptidsequenzen, die z.B. aus der Galactosidase von *E. coli* abgeleitet sein können. Weiterhin können auch virale Peptidsequenzen, wie zum Beispiel vom Bakteriophagen M13 verwendet werden, um so Fusionsproteine für das dem Fachmann bekannte "phage display"-Verfahren zu erzeugen.

Weitere bevorzugte Beispiele für Peptidsequenzen für erfindungsgemäß verwendbare Fusionsproteine sind Peptide, die die Detektion der Fusionsproteine erleichtem. Hierzu zählen beispielsweise "Green-fluorescent-protein" (WO 95/07463).

Zur Aufreinigung der vorangehend beschriebenen Proteine kann ein weiteres/weiterer Polypeptid ("tag") angefügt sein. Erfindungsgemäße Protein-tags erlauben beispielsweise die hochaffine Adsorption an eine Matrix, stringentes Waschen mit geeigneten Puffern, ohne den Komplex in nennenswertem Maße zu eluieren und anschließend gezielte Elution des adsorbierten Komplexes. Beispiele der dem Fachmann bekannten Protein-tags sind ein (His)₆-tag, ein Myc-tag, ein FLAG-tag, ein Hämagglutinin-tag, Glutathion-Transferase (GST)-tag, Intein mit einem Affinitäts-Chitin-binding-tag oder Maltose-binding protein (MBP)-tag. Diese Protein-tags können sich N-, C-terminal und/oder intern befinden.

Der Begriff "kodierende Nukleinsäure" bezieht sich auf eine DNA-Sequenz gemäß SEQ ID Nr. 2 und SEQ ID Nr. 4, die für ein isolierbares funktionelles erfindungsgemäßes Polypeptid oder einen Vorläufer, z.B. mit einer Signalsequenz, kodiert. Das Polypeptid kann durch eine Sequenz in voller Länge oder jeden Teil der kodierenden Sequenz kodiert werden, solange die spezifische, beispielsweise enzymatische Aktivität erhalten bleibt.

Es ist bekannt, dass Veränderungen in der Sequenz der vorangehend beschriebenen Nukleinsäuren vorhanden sein können, zum Beispiel durch die Degenerierung des genetischen Codes oder dass nicht translatierte Sequenzen beispielsweise am 5'- und/oder 3'-Ende der Nukleinsäure angehängt sein können, ohne dass deren Aktivität wesentlich verändert wird. Auch können die unten näher beschriebenen Modifikationen durchgeführt werden. Diese Erfindung beschreibt deshalb auch sogenannte "Varianten" der vorangehend beschriebenen Nukleinsäuren.

Unter "Varianten" der Nukleinsäuren sind alle DNA-Sequenzen zu verstehen, die komplementär zu einer DNA-Sequenz sind, die unter stringenten Bedingungen mit der Referenzsequenz hybridisieren und eine im Wesentlichen gleiche Aktivität aufweisen wie das von der Referenzsequenz kodierte Polypeptid.

Unter "stringenten Hybridisierungsbedingungen" sind solche Bedingungen zu verstehen, bei denen eine Hybridisierung beispielsweise bei 60°C in 2,5 x SSC-Puffer, gefolgt von mehreren Waschschritten bei 37°C in einer geringeren Pufferkonzentration erfolgt, und stabil bleibt.

Varianten der Nukleinsäure können auch Teile der erfindungsgemäß verwendeten Nukleinsäure mit mindestens 8 Nukleotiden Länge, vorzugsweise mit mindestens 18 Nukleotiden Länge, insbesondere mit mindestens 24 Nukleotiden Länge, besonders bevorzugt mit mindestens 30 Nukleotiden, vorzugsweise bevorzugt mit mindestens 42 Nukleotiden sein.

Bevorzugterweise handelt es sich bei den erfindungsgemäß verwendbaren Nukleinsäure um DNA oder RNA, vorzugsweise eine DNA, besonders bevorzugt eine doppelsträngige DNA. Weiterhin kann die Sequenz der Nukleinsäuren dadurch gekennzeichnet sein, dass sie mindestens ein Intron und/oder eine polyA-Sequenz aufweist. Die erfindungsgemäß verwendeten Nukleinsäuren können auch in Form ihrer antisense-Sequenz vorliegen.

Weiterhin kann zur Durchführung der Erfindung eine Nukleinsäure verwendet werden, die synthetisch hergestellt worden ist. So kann die erfindungsgemäß verwendete Nukleinsäure beispielsweise chemisch anhand der in Tabelle 1 beschriebenen DNA Sequenzen und/oder anhand der in Tabelle 1 ebenfalls beschriebenen Proteinsequenzen unter Heranziehen des genetischen Codes z.B. nach der Phosphotriester-Methode synthetisiert werden (siehe z.B. Uhlmann, E. & Peyman, A. (1990) Chemical Reviews 90:543-584).

Unter "Expression" im Sinne der vorliegenden Erfindung ist die in einem bestimmten Zelltyp vorhandene Menge an FGF-BP Polypeptiden und/oder FGF-BP kodierenden Nukleinsäuren zu verstehen.

Unter dem Begriff "Regulation" wird beispielsweise die Erhöhung oder Erniedrigung der Polypeptidmenge oder diese kodierende Nukleinsäure verstanden, wobei diese Änderung beispielsweise auf transkriptioneller, translationeller, posttranskriptioneller und posttranslationeller Ebene stattfinden kann.

Für die Expression des betreffenden Gens ist im Allgemeinen eine doppelsträngige DNA bevorzugt, wobei der für das Polypeptid kodierende DNA-Bereich besonders bevorzugt ist. Dieser Bereich beginnt bei Eukaryonten mit dem ersten in einer Kozak Sequenz (Kozak, 1987, Nucleic. Acids Res. 15:8125-48) liegenden Start-Codon (ATG) bis zum nächsten Stop-Codon (TAG, TGA bzw. TAA), das im gleichen Leseraster zum ATG liegt. Bei Prokaryonten beginnt dieser Bereich mit einer ein paar Nukleotide oberhalb des Start-Codon liegenden konservierten Sequenz von 6 Nukleotiden, die sogenannte Shine-Dalgarno Sequenz (Shine & Dalgarno, 1975, Eur. J. Biochem. 57:221-30).

Expressionsvektoren können prokaryotische oder eukaryotische Expressionsvektoren sein. Beispiele für prokaryotische Expressionsvektoren sind für die Expression in *E. coli* z.B. die Vektoren pGEM oder pUC-Derivate und für eukaryotische Expressionsvektoren für die Expression in *Saccharomyces cerevisiae* z.B. die Vektoren p426Met25 oder p426GAL1 (Mumberg et al. (1994) Nucl. Acids Res. 22:5767-5768), für die Expression in Insektenzellen z.B. *Baculovirus*-Vektoren wie in EP-B1-0 127 839 oder EP-B1-0 549 721 offenbart, und für die Expression in Säugerzellen z.B. die Vektoren Rc/CMV und Rc/RSV oder SV40-Vektoren, welche alle allgemein erhältlich sind.

Im Allgemeinen enthalten die Expressionsvektoren auch für die jeweilige Zelle geeignete Promotoren, wie z.B. den trp-Promotor für die Expression in *E. coli* (siehe z.B. EP-B1-0 154 133), den Met 25, GAL 1 oder ADH2-Promotor für die Expression in Hefen (Russel et al. (1983) J. Biol. Chem. 258:2674-2682; Mumberg, supra) und den Baculovirus-Polyhedrin-Promotor für die Expression in Insektenzellen (siehe z. 13. EP-B1-0 127 839). Für die Expression in Säugetierzellen sind beispielsweise Promotoren geeignet, die eine konstitutive, regulierbare, gewebespezifische, zellzyklusspezifische oder Metabolismus-spezifische Expression in eukaryotischen Zellen erlauben. Regulierbare Elemente gemäß der vorliegenden Erfindung sind Promotoren, Aktivatorsequenzen, Enhancer, Silencer und/oder Repressorsequenzen.

Beispiele für geeignete Elemente, die konstitutive Expression in Eukaryonten ermöglichen, sind Promotoren, die von der RNA Polymerase III erkannt werden oder virale Promotoren, CMV-Enhancer, CMV-Promotor, SV40-Promotor oder LTR-Promotoren z.B. von MMTV (mouse mammary tumour virus; Lee et al. (1981) Nature 214:228-232) und weitere virale Promotor- und Aktivatorsequenzen, abgeleitet aus beispielsweise HBV, HCV, HSV, HPV, EBV, HTLV oder HIV.

Vorzugsweise erfolgt die Expression von wundheilungsrelevanten Genen unter der Kontrolle von gewebespezifischen Promotoren, wobei hautspezifische Promotoren wie beispielsweise der humane K10 Promotor (Bailleul et al. (1990) Cell 62:697-708), der humane K14 Promotor (Vassar et al. (1989) Proc. Natl. Acad. Sci. USA 86:1563-67) oder der bovine Cytokeratin IV Promotor (Fuchs et al. (1988) The biology of wool and hair (Hrsg.: G.E. Rogers, et al.), S. 287-309. Chapman und Hall, London/New York) besonders zu bevorzugen sind.

Weitere Beispiele für Elemente, die gewebespezifische Expression in Eukaryonten ermöglichen, sind Promotoren oder Aktivatorsequenzen aus Promotoren oder Enhancern von solchen Genen, die für Proteine kodieren, die nur in bestimmten Zelltypen konstitutiv exprimiert werden.

Beispiele für Elemente, die regulierbare Expression in Eukaryonten ermöglichen, sind der Tetracyclinoperator in Kombination mit einem entsprechenden Repressor (Gossen M. et al. (1994) Curr. Opin. Biotechnol. 5:516-20).

Beispiele für regulierbare Elemente, die zellzyklusspezifische Expression in Eukaryonten ermöglichen, sind Promotoren folgender Gene: cdc25A, cdc25B, cdc25C, Cyclin A, Cyclin E, cdc2, E2F-1 bis E2F-5, B-myb oder DHFR (Zwicker J. und Müller R. (1997) Trends Genet. 13, 3-6). Die Verwendung von zellzyklusregulierten Promotoren ist besonders bevorzugt in Fällen, in denen die Expression der erfindungsgemäß verwendeten Polypeptide oder Nukleinsäuren auf proliferierende Zellen beschränkt werden soll.

Beispiele für regulierbare Elemente, die Metabolismus-spezifische Expression in Eukaryonten ermöglichen, sind Promotoren, die durch Hypoxie, durch Glukosemangel, durch Phosphatkonzentration oder durch Hitzeschock reguliert werden.

Beispiele für regulierbare Elemente, die gleichzeitig eine räumlich und zeitlich begrenzte Expression erlauben, sind Nukleinsäuren, die für eine Fusion zwischen der Sequenz für die ortsspezifische Rekombinase Cre und einem modifizierten Östrogenrezeptor unter der Kontrolle eines gewebespezifischen Promotors kodieren. Das resultierende, gewebespezifische zytoplasmatische Fusionsprotein kann durch Gabe des Östrogenanalogs Tamoxifen in den Zellkern translozieren und Rekombinationen erzeugen, die zu veränderter Genexpression führen (Feil et al. (1996) Proc. Natl. Acad. Sci. 93:10887-90).

Um die Einführung der vorangehend beschriebenen Nukleinsäuren und damit die Expression des FGF-BP Polypeptids in einer eu- oder prokaryotischen Zelle durch Transfektion, Transformation oder Infektion zu ermöglichen, kann die für FGF-BP kodierende Nukleinsäure gemäß SEQ ID Nr. 2 und 4 oder funktionelle Varianten hiervon in Form eines Expressionsvektors, insbesondere eines gentherapeutisch wirksamen Vektors eingesetzt werden. In weiteren Beispielen kann die für FGF-BP kodierende Nukleinsäure in einem "shuttle" Vektor, Phagemid, Cosmid, Plasmid oder als Teil eines viralen oder nicht-viralen Vektors vorliegen. Als virale Vektoren eignen sich hierbei besonders: Baculoviren, Vakziniaviren, Adenoviren, adenoassoziierte Viren und Herpesviren. Als nicht-virale Vektoren eignen sich hierbei besonders: Virosomen, Liposomen, kationische Lipide, oder poly-Lysin konjugierte DNA.

Vorzugsweise enthält ein gentherapeutisch wirksamer Vektor wund- bzw. hautspezifische regulatorische Sequenzen, die funktionell mit der vorangehend beschriebenen Nukleinsäure verbunden sind.

Für die gentherapeutische Anwendung der vorangehend beschriebenen Nukleinsäure ist es auch von Vorteil, wenn der Teil der Nukleinsäure, der für das Polypeptid kodiert, ein oder mehrere nicht kodierende Sequenzen einschließlich Intronsequenzen, vorzugsweise zwischen Promotor und dem Startcodon des Polypeptids, und/oder eine polyA-Sequenz, insbesondere die natürlich vorkommende polyA-Sequenz oder eine SV40 Virus polyA-Sequenz, vor allem am 3'-Ende des Gens enthält, da hierdurch eine Stabilisierung der mRNA erreicht werden kann (Beispiel 3; Palmiter et al. (1991) Proc. Natl. Acad. Sci. USA 88:478-482 ; Jackson (1993) Cell 74:9-14).

Unter einem "gentherapeutischen Vektor" im Sinne der vorliegenden Erfindung, ist eine Nukleinsäure zu verstehen, in die eine erfindungsgemäß verwendbare Nukleinsäure integriert werden kann und die anschließend in eine Zelle eingeschleust wird und in dieser die fehlende oder defekte Expression der erfindungsgemäßen Nukleinsäure ergänzt oder ersetzt.

Beispiele von gentherapeutisch wirksamen Vektoren sind Virusvektoren, beispielsweise Adenovirusvektoren oder retroviralen Vektoren (Lindemann et al. (1997), Mol. Med. 3:466-76; Springer et al. (1998) Mol. Cell. 2:549-58). Eukaryotische Expressionsvektoren eignen sich in isolierter Form für die gentherapeutische Anwendung, da nackte DNA bei topischer Applikation in Hautzellen eindringen kann (Hengge et al. (1996) J. Clin. Invest. 97:2911-6; Yu et al. (1999) J. Invest. Dermatol. 112:370-5).

Gentherapeutisch wirksame Vektoren lassen sich auch dadurch erhalten, dass man die vorangehend beschriebenen Nukleinsäuren mit Liposomen komplexiert, da damit eine sehr hohe Transfektionseffizienz, insbesondere von Hautzellen, erreicht werden kann (Alexander und Akhurst, 1995, Hum. Mol. Genet. 4:2279-85). Bei der Lipofektion werden kleine unilamellare Vesikel aus kationischen Lipiden durch Ultraschallbehandlung der Liposomensuspension herstellt. Die DNA wird ionisch auf der Oberfläche der Liposomen gebunden, und zwar in einem solchen Verhältnis, dass eine positive Nettoladung verbleibt und die Plasmid-DNA zu 100% von den Liposomen komplexiert wird. Neben den von Felgner et al. (1987, supra) eingesetzten Lipidmischungen DOTMA (1,2-Dioleyloxpropyl-3-trimethylammoniumbromid) und DOPE (Dioleoylphosphatidylethanolamin) wurden inzwischen zahlreiche neue Lipidformulierungen synthetisiert und auf ihre Effizienz bei der Transfektion verschiedener Zelllinien getestet (Behr et al. (1989) Proc. Natl. Acad. Sci. USA 86:6982-6986; Gao und Huang (1991), Biochim. Biophys. Acta 1189:195-203; Felgner et al. (1994) J. Biol. Chem. 2692550-2561). Beispiele der neuen Lipidformulierungen sind DOTAP N-[1-(2,3-Dioleoyloxy)propyl]-N,N,N-trimethylammoniumethylsulfat oder DOGS (TRANSFECTAM; Dioctadecylamidoglycylspermin). Hilfsstoffe, die den Transfer von Nukleinsäuren in die Zelle erhöhen, können beispielsweise Proteine oder Peptide, die an DNA gebunden sind oder synthetische Peptid-DNA-Moleküle, die den Transport der Nukleinsäure in den Kern der Zelle ermöglichen, sein (Schwartz et al. (1999) Gene Therapy 6:282; Branden et al. (1999) Nature Biotech. 17:784). Hilfsstoffe umfassen auch Moleküle, die die Freisetzung von Nukleinsäuren in das Zytoplasma der Zelle ermöglichen (Planck et al. (1994) J. Biol. Chem. 269:12918; Kichler et al. (1997) Bioconj. Chem. 8:213) oder beispielsweise Liposomen (Uhlmann und Peymann (1990) supra).

Eine andere besonders geeignete Form von gentherapeutischen Vektoren lässt sich dadurch erhalten, dass man die vorangehend beschriebene Nukleinsäure auf Goldpartikeln aufbringt und diese mit Hilfe der sogenannten "Gene Gun" in Gewebe, bevorzugt in die Haut, oder Zellen schießt (Wang et al. (1999) J. Invest. Dermatol. 112:775-81, Tuting et al. (1998) J. Invest. Dermatol. 111:183-8).

Eine weitere Ausführungsform eines gentherapeutisch wirksamen erfindungsgemäß verwendbaren Vektors lässt sich durch das Einbringen von "nackten" Expressionsvektoren in eine biokompatible Matrix, beispielsweise eine Kollagen-matrix, herstellen. Diese Matrix kann in Wunden eingebracht werden, um die einwandernden Zellen mit dem Expressionsvektor zu transfizieren und die erfindungsgemäßen Polypeptide in den Zellen zu exprimieren (Goldstein und Banadio, US 5,962,427).

Die Therapie von diabetes-assoziierten Wundheilungsstörungen, insbesondere diabetes-assoziierten Wunden, kann auf herkömmliche Weise, z.B. durch Verbände, Pflaster, Kompressen oder Gele erfolgen, die die erfindungsgemäßen Arzneimittel enthalten. So ist es möglich, die geeignete Zusatz- oder Hilfsstoffe, wie z.B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs etc., enthaltenden Arzneimittel topisch und lokal zu verabreichen, um die Wundheilung sofort und unmittelbar zu beeinflussen. Die Verabreichung der erfindungsgemäßen Arzneimittel kann weiterhin gegebenenfalls in Form von Liposomenkomplexen bzw. Goldpartikelkomplexen ebenfalls topisch und lokal im Bereich der Wunde erfolgen. Weiterhin kann die Behandlung mittels eines transdermalen therapeutischen Systems (TTS) erfolgen, das eine zeitlich gesteuerte Abgabe der erfindungsgemäß verwendbaren Arzneimittel ermöglicht. Die Behandlung mittels der erfindungsgemäß verwendbaren Arzneimittel kann aber auch über orale Dosierungsformen, wie z.B. Tabletten oder Kapseln, über die Schleimhäute, zum Beispiel der Nase oder der Mundhöhle, oder in Form von unter die Haut implantierten Dispositorien erfolgen. TTS sind zum Beispiel aus den EP 0 944 398, EP 0 916 336, EP 0 889 723 oder EP 0 852 493 bekannt.

Für die gentherapeutische Anwendung beim Menschen ist vor allem ein Arzneimittel geeignet, das die beschriebenen Nukleinsäuren in nackter Form oder in Form eines der oben beschriebenen gentherapeutisch wirksamen Vektoren oder in mit Liposomen bzw. Goldpartikeln komplexierter Form enthält. Der pharmazeutische Träger ist beispielsweise eine physiologische Pufferlösung, vorzugsweise mit einem pH von ca. 6,0-8,0, vorzugsweise von ca. 6,8-7,8, insbesondere von ca. 7,4 und/oder einer Osmolarität von ca. 200-400 milliosmol/Liter, vorzugsweise von ca. 290-310 milliosmol/Liter. Zusätzlich kann der pharmazeutische Träger geeignete Stabilisatoren, wie z.B. Nukleaseinhibitoren, vorzugsweise Komplexbildner wie EDTA und/oder andere dem Fachmann bekannte Hilfsstoffe enthalten.

Die Verabreichung der beschriebenen Nukleinsäuren gegebenenfalls in Form der oben näher beschriebenen Virusvektoren oder als Liposomenkomplexe bzw. Goldpartikelkomplex erfolgt üblicherweise topisch und lokal im Bereich der Wunde.

Eine bevorzugte Ausführungsform eines Arzneimittels gemäß der vorliegenden Erfindung besteht darin, das Polypeptid selbst mit geeigneten Zusatz- oder Hilfsstoffen, wie z.B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., zu verabreichen, um die Wundheilung sofort und unmittelbar zu beeinflussen (Beispiel 4). Diese Ausführungsform ist besonders vielversprechend bei vorliegender Erfindung, da es sich beim FGF-BP Polypeptid um ein sezerniertes Protein handelt.

Eine weitere bevorzugte Ausführungsform eines Arzneimittels gemäß vorliegender Erfindung besteht in der Verwendung eines gegen ein erfindungsgemäß verwendbares Polypeptid gerichteten Antikörpers, der die Aktivität des FGF-BP steigert oder unterstützt. Beispielsweise könnte ein solcher Antikörper die Freisetzung von FGF positiv beeinflussen und zur Behandlung und Prävention von Wundheilungsstörungen, die durch eine verringerte Menge an FGF-BP gekennzeichnet sind, beitragen.

Eine weitere bevorzugte Ausführungsform eines Arzneimittels gemäß der vorliegenden Erfindung besteht in der Transplantation autologer oder allogener Zellen, die das erfindungsgemäße FGF-BP Polypetid exprimieren und sezemieren, mit Hilfe eines geeigneten Trägermaterials, z.B. sogenannte Microcarrier, die aus biokompatiblen Materialien, wie beispielsweise einer Dextranmatrix, bestehen (US 5,980,888).

Ein weitere bevorzugte Ausführungsform der vorliegenden Erfindung ist die Verwendung einer Zelle, die mit einem erfindungsgemäß verwendbaren Vektor oder knock-out Genkonstrukt zur Diagnose und/oder Prävention von diabetes-assoziierte Wunden, sowie zur Identifizierung von pharmakologisch aktiven Substanzen transformiert ist. Zellen können sowohl prokaryotische als auch eukaryotische Zellen sein, Beispiele für prokaryotische Zellen sind *E. coli* und für eukaryotische Zellen *Saccharomyces cerevisiae* oder Insektenzellen.

Zellen, die ein erfindungsgemäßes FGF-BP Polypeptid oder gegen erfindungsgemäße FGF-BP Polypeptide gerichtete Antikörper oder Antikörperfragmente exprimieren sind z.B. humane, nicht-embryonale, autologe oder allogene Zellen. Bevorzugt handelt es sich bei den genannten Zellen um Hautzellen, insbesondere Keratinozyten, Fibroblasten oder Endothelzellen.

Ein besonders bevorzugte erfindungsgemäß verwendbare transformierte Zelle ist eine transgene embryonale nicht-menschliche Stammzelle, die dadurch gekennzeichnet ist, dass sie mindestens ein erfindungsgemäß verwendbares knock-out Genkonstrukt und/oder mindestens eine erfindungsgemäß verwendbare Expressionskassette, wie vorangehend beschrieben, enthält. Verfahren zur Transformation von Zellen und/oder Stammzellen sind dem Fachmann gut bekannt und umfassen zum Beispiel Elektroporation oder Mikroinjektion.

Eine weitere bevorzugte Ausführungsform eines Arzneimittels gemäß vorliegender Erfindung betrifft die Verwendung mindestens eines FGF-BP Polypeptids gemäß SEQ ID Nr. 1 und 3 und/oder mindestens einer für FGF-BP kodierenden Nukleinsäure gemäß SEQ ID Nr. 2 und 4 oder gegen ein erfindungsgemäß verwendbares FGF-BP Polypeptid gerichteter Antikörper oder Antikörperfragmente und/oder mindestens einer FGF-BP Polypeptid exprimierenden Zelle zur in vitro Identifizierung pharmakologisch aktiver Substanzen, die die Funktion und/oder die Expression des FGF-BP Polypeptids und/oder einer für FGF-BP Polypeptid kodierenden Nukleinsäure steigern zur Behandlung und/oder Prävention von diabetes-assoziierten Wunden. Die genannten pharmakologisch aktiven Substanzen können organische und anorganische Substanzen sein, die die Aktivität und/oder die Expression des FGF-BP Proteins und/oder DNA modulieren, insbesonderte aktivieren. Die Verwendung dieser organischen oder anorganischen pharmakologisch aktiven Substanzen als Arzneimittel kann durch Applikation wie oben beschrieben erfolgen. Beispiele solcher aktivierender Substanzen finden sich bei Harris et al. (2000) J. Biol. Chem. 275:10802-10811.

Die vorliegende Erfindung betrifft weiterhin die Verwendung mindestens eines FGF-BP Polypeptids gemäß SEQ ID Nr. 1 und 3 und/oder mindestens einer für FGF-BP kodierenden Nukleinsäure gemäß SEQ ID Nr. 2 und 4 oder gegen ein erfindungsgemäß verwendbares FGF-BP Polypeptid gerichteter Antikörper oder Antikörperfragmente und/oder mindestens einer FGF-BP Polypeptid exprimierenden Zelle, gegebenenfalls kombiniert oder zusammen mit geeigneten Zusatz- und Hilfsstoffen, zur Herstellung eines Tests zur Auffindung von pharmakologisch aktiven Substanzen in Zusammenhang mit diabetes-assoziierten Wunden.

Unter dem Begriff "pharmakologisch aktive Substanzen" im Sinne der vorliegenden Erfindung sind alle diejenigen Moleküle, Verbindungen und/oder Zusammensetzungen und Stoffgemische zu verstehen, die mit den vorangehend beschriebenen Nukleinsäuren, Polypeptiden, Antikörpern oder Antikörperfragmenten, gegebenenfalls zusammen mit geeigneten Zusatz- und Hilfsstoffen, unter geeigneten Bedingungen in Wechselwirkung treten können. Mögliche pharmakologisch aktive Substanzen sind einfache chemische organische oder anorganische Moleküle oder Verbindungen, können aber auch Nukleinsäuren, Peptide, Proteine oder Komplexe davon umfassen. Die pharmakologisch aktiven Substanzen können aufgrund ihrer Wechselwirkung die Funktion(en) der Nukleinsäuren, Polypeptide oder Antikörper *in vivo* oder *in vitro* beeinflussen oder auch nur an die vorangehend beschriebenen Nukleinsäuren, Polypeptide, Antikörper oder Antikörperfragmente binden oder mit ihnen andere Wechselwirkungen kovalenter oder nichtkovalenter Weise eingehen. Beispiele pharmakologisch aktiver Substanzen sind weiterhin organische Moleküle, die Substanzbibliotheken entstammen, die auf ihre pharmakologische Aktivität hin überprüft wurden.

Bevorzugte pharmakologisch aktive Substanzen gemäß vorliegender Erfindung sind solche, die aktivierend wirken.

In einer Ausführungsform umfassen die Tests zur Auffindung von pharmakologisch aktiven Substanzen des erfindungsgemäßen FGF-BP Assays, die die Expression von Genen beeinflussen und dem Fachmann allgemein bekannt sind (siehe beispielsweise Sivaraja et al. (2001) US 6,183,956). So können Zellen, die FGF-BP exprimieren, beispielsweise Hautzellen, als Testsystem zur Analyse der Genexpression *in vitro* kultiviert werden, wobei Keratinozyten, Fibroblasten oder Endothelzellen zu bevorzugen sind. Ein mögliches Testsystem stellt dabei die menschliche Keratinozyten-Zelllinie HaCaT dar, die allgemein erhältlich ist. Die Analyse der Genexpression erfolgt beispielsweise auf der Ebene der mRNA oder der Proteine. Dabei wird die Menge an FGF-BP mRNA und/oder Protein nach Zugabe einer oder mehrerer Substanzen zur Zellkultur gemessen und mit der entsprechenden Menge in einer Kontrollkultur verglichen. Dies geschieht beispielsweise mit Hilfe von Hybridisierung einer antisense Sonde, mit der im Lysat der Zellen enthaltenen mRNA eines erfindungsgemäßen FGF-BP. Dabei kann die Hybridisierung beispielsweise über die Bindung eines spezifischen Antikörpers an den mRNA-Sondenkomplex quantifiziert werden (siehe Stuart & Frank, 1998, US 4,732,847). Dabei ist es möglich, die Analyse im Hochdurchsatzverfahren durchzuführen und sehr viele Substanzen auf ihre Eignung als Modulator der Expression von FGF-BP zu analysieren (Sivaraja et al. (2001) US 6,183,956). Die zu analysierenden Substanzen können dabei aus Substanzbibliotheken (siehe z.B. DE 19816414, DE 19619373) entnommen werden, die mehrere tausend, oft sehr heterogene Substanzen enthalten können. Alternativ kann zunächst die Gesamt-RNA oder -mRNA aus Zellen isoliert werden und anschließend die absolute Menge oder der relative Anteil der mRNA von FGF-BP beispielsweise mit Hilfe der quantitativen RT-PCR (siehe EP 0 200 362; Wittwer , 1997, Bio Techniques 22:130-138; Morrison et al. (1998) Bio Techniques 24:954-62) oder des RNase Protection Assays (siehe z.B. Sambrook et al. (1989) Molecular Cloning:A Laboratary Manual, Cold Spring Harbor Laboratory, Cold Spring Harbor Laboratory Press, New York, Kapitel 7; EP 0 063 879) bestimmt werden. Eine weitere Möglichkeit stellt die Analyse der Proteinmenge im Zelllysat mit Antikörpern dar, die spezifisch FGF-BP erkennen. Hier kann die Quantifizierung beispielsweise mit Hilfe eines ELISAs oder einer Western Blots erfolgen, die allgemein bekannt sind. Um die Spezifität der Substanzen auf die Expression von FGF-BP zu vergleichen, kann der Einfluss der Substanzen auf die Expression des FGF-BP mit dem Einfluss auf die Expression anderer Gene, wie beispielsweise Gene des Stoffwechsels wie GAPDH oder Cyclophilin, verglichen werden. Besonders zu bevorzugen sind Assays, die geeignet sind Substanzen zu identifizieren, die die Expression von einem oder mehreren Kontrollgenen, wie beispielsweise GAPDH oder Cyclophilin, möglichst wenig beeinflussen (siehe z.B. WO 00/05416).

Eine weitere Ausführung vorliegender Erfindung betrifft die Verwendung von FGF-BP Polypeptiden zur in vitro Identifizierung pharmakologisch aktiver Substanzen, die die Aktivität von FGF-BP erhöhen, mit Hilfe von Screening-Verfahren. So gibt es beispielsweise das sogenannten "Two-Hybrid System" (Fields und Sternglanz, 1994, Trends in Genetics, 10, 286-292; Colas und Brent, 1998 TIBTECH, 16, 355-363). Bei diesem Test werden Zellen mit Expressionsvektoren transformiert, die Fusionsproteine aus dem erfindungsgemäßen Polypeptid und einer DNA-Bindungsdomäne eines Transkriptionsfaktors wie beispielsweise Gal4 oder LexA exprimieren. Die transformierten Zellen enthalten außerdem ein Reportergen, dessen Promotor Bindungsstellen für die entsprechende DNA Bindungsdomäne enthalten. Durch Transformation eines weiteren Expressionsvektors, der ein zweites Fusionsprotein aus einem bekannten bzw. unbekannten Polypeptid mit einer Aktivierungsdomäne, beispielsweise von Gal4 oder Herpes simplex Virus VP16 exprimiert, kann die Expression des Reportergens stark gesteigert werden, wenn das zweite Fusionsprotein mit dem erfindungsgemäßen Polypeptid interagiert. Diese Expressionssteigerung kann man ausnutzen, um neue pharmakologisch aktive Substanzen zu identifizieren, beispielsweise indem man zur Konstruktion des zweiten Fusionsproteins eine cDNA-Bibliothek aus sich regenerierundem Gewebe herstellt. Zudem lässt sich dieses Testsystem zur Rasteruntersuchung von Substanzen ausnutzen, die eine Interaktion zwischen dem erfindungsgemäßen Polypeptid und einem Interaktor inhibieren. Solche Substanzen verringern die Expression des Reportergens in Zellen, die Fusionsproteine des erfindungsgemäßen Polypeptids und der pharmakologisch aktiven Substanz exprimieren (Vidal und Endoh, 1999, Trends in Biotechnology, 17:374-81). So lassen sich schnell neue Wirkstoffe identifizieren, die zur Therapie von Störungen regenerativer Prozesse eingesetzt werden können. Die erfolgreiche Verwendung des erfindungsgemäßen FGF-BP in "Two-Hybrid"- Systemen wurde durch das "Fischen" des FGF-BP aus einer Keratinozyten -Bibliothek mit Perlecan als "Köder" bereits gezeigt (Mongiat et al, 2001, J. Biol. Chem. 276:10263-10271).

Ein bevorzugte Ausführungsform dieser erfindungsgemäß verwendbaren Testsysteme besteht in der Verwendung von z.B. HaCaT-Zellen, die allgemein erhältlich sind, und des Expressionsvektors pCMV4 (Anderson et al. (1989) J. Biol. Chem. 264:8222-9). Eine FGF-BP kodierende Nukleinsäure kann dabei sowohl in sense als auch in antisense Orientierung in die Expressionsvektoren integriert werden, so dass die funktionelle Konzentration an mRNA der entsprechenden Gene in den Zellen entweder erhöht, oder durch Hybridisierung mit der antisense-RNA erniedrigt wird. Nach der Transformation und Selektion stabiler Transformanden zeigen die Zellen in Kultur im Allgemeinen ein verändertes Proliferations-, Migrationsund/oder Differenzierungsverhalten im Vergleich zu Kontrollzellen. Dieses Verhalten *in vitro* ist häufig mit der Funktion der entsprechenden Gene bei regenerativen Prozessen im Organismus korreliert (Yu et al. (1997) Arch. Dermatol. Res. 289:352-9; Mils et al. (1997) Oncogene 14:15555-61; Charvat et al. (1998) Exp. Dermatol. 7:184-90; Werner (1998) Cytokine Growth Factor Rev. 9:153-65; Mythily et al. (1999) J. Gen. Virol. 80:1707-13) und lässt sich mit einfachen und schnell durchzuführenden Tests nachweisen, so dass man darauf basierend Testsysteme für pharmakologisch aktive Substanzen aufbauen kann. So lässt sich das Proliferationsverhalten von Zellen sehr schnell durch z.B. den Einbau von markierten Nukleotiden in die DNA der Zellen (siehe z.B. Savino und Dardenne, 1985, J. Immunol. Methods 85:221-6; Perros und Weightman, 1991, Cell Prolif. 24:517-23; de Fries und Mitsuhashi, 1995, J. Clin. Lab. Anal. 9:89-95), durch Anfärbung der Zellen mit spezifischen Farbstoffen (Schulz et al. (1994) J. Immunol. Methods 167:1-13) oder über immunologische Verfahren (Frahm et al. (1998) J. Immunol. Methods 211:43-50) nachweisen. Die Migration lässt sich einfach durch den "Migration Index" Test (Charvat et al., supra) und vergleichbare Testsysteme (Benestad et al. (1987) Cell Tissue Kinet. 20:109-19, Junger et al. (1993) J. Immunol. Methods 160:73-9) nachweisen. Als Differenzierungsmarker eignen sich z.B. Keratin 6, 10 und 14 sowie Loricrin und Involucrin (Rosenthal et al. (1992) J. Invest. Dermatol. 98:343-50), deren Expression z.B. über allgemein erhältliche Antikörper leicht nachzuweisen ist. Die auf diese Weise identifizierten Veränderungen der Zellaktivitäten gegenüber einem Kontrollexperiment, bei dem Zellen beispielsweise mit einem leeren Expressionsvektor transfiziert wurden, können genutzt werden, um die Effizienz von Modulatoren zu untersuchen.

Weiterhin kann ein Testsystem darauf beruhen, dass mindestens ein erfindun^{g}sgemäß verwendbares FGF-BP Polypeptid oder diese kodierende Nukleinsäuren, oder gegen erfindungsgemäß verwendbare Polypeptide gerichtete Antikörper oder Antikörperfragmente an eine Festphase gebunden sind und mindestens eine Substanz auf ihre pharmakologische Aktivität, beispielsweise Bindung oder Änderung der Konformation, hin untersucht wird. getestet werden. Geeignete Systeme wie die Affinitätschromatographie und Fluoreszenzspektroskopie sind dem Fachmann bekannt. So konnte eine Bindung zwischen einem erfindungsgemäßen FGF-BP und Heparin mit Hilfe von Heparin-Kupfer Affinitätschromatopraphie bereits erfolgreich gezeigt werden (Wang et al. (1998) Biochem. Mol. Biol. Int. 46:81-87).

Die Festphasen-gebundenen erfindungsgemäß verwendbaren Polypeptide oder diese kodierende Nukleinsäuren oder gegen ein erfindungsgemäß verwendbares Polypeptid gerichtete Antikörper oder Antikörperfragmente können auch Teil eines Arrays sein. Verfahren zur Herstellung von solchen Arrays mittels Festphasenchemie und photolabilen Schutzgruppen sind zum Beispiel aus der US 5,744,305 bekannt. Diese Arrays können ebenfalls mit Substanzen oder Substanzbibliotheken in Kontakt gebracht werden und auf Interaktion, beispielsweise Bindung oder Konformationsänderung, getestet werden.

So kann beispielsweise eine zu testende Substanz einen detektierbaren Marker enthalten, beispielsweise kann die Substanz radioaktiv markiert, Fluoreszenz-markiert oder Lumineszenz-markiert sein. Weiterhin können Substanzen an Proteine gekoppelt werden, die eine indirekte Detektion erlauben, beispielsweise über enzymatische Katalyse mittels eines Peroxidase-Assays mit einem chromogenen Substrat oder durch Bindung eines detektierbaren Antikörpers. Eine weitere Möglichkeit besteht darin, die Array-gebundenen Proteinkomplexe per Massenspekrometrie zu untersuchen (SELDI). Änderungen der Konformation eines erfindungsgemäß verwendbaren Polypeptids durch Interaktion mit einer Testsubstanz können beispielsweise durch Änderung der Fluoreszenz eines endogenen Tryptophanrestes im Polypeptid nachgewiesen werden. So konnte mit Hilfe von SELDI die Interaktion des erfindungsgemäßen FGF-BP mit bFGF nachgewiesen und anschießend in einem radioaktiven ELISA quantifiziert werden (Tassi et al. (2001) J. Biol. Chem. 276:40247-53).

Pharmakologisch aktive Substanzen der erfindungsgemäß verwendbaren Polypeptide können auch Nukleinsäuren sein, die über Selektionsverfahren, wie beispielsweise SELEX (siehe Jayasena, 1999, Clin. Chem. 45:1628-50; Klug und Famulok, 1994, M. Mol. Biol. Rep. 20:97-107; Toole et al. (1996) US 5,582,981) isoliert werden. Im SELEX-Verfahren werden typischerweise aus einem großen Pool unterschiedlicher, einzelsträngiger RNA Moleküle durch wiederholte Amplifikation und Selektion diejenigen Moleküle isoliert, die an ein erfindungsgemäß verwendbares Polypeptid mit hoher Affinität binden (Aptamere). Aptamere können auch in ihrer spiegelbildlichen Form, beispielsweise als L-Ribonukleotid, synthetisiert und selektioniert werden (Nolte et al. (1996) Nat. Biotechnol. 14:1116-9; Klussmann et al. (1996) Nat. Biotechnol. 14:1112-5). So isolierte Formen haben den Vorteil, dass sie nicht von natürlich vorkommenden Ribonukleasen abgebaut werden und daher größere Stabilität besitzen.

Oligonukleotide werden in der Regel schnell durch Endo- oder Exonukleasen, insbesondere durch in der Zelle vorkommende DNasen und RNasen, abgebaut. Deshalb ist es vorteilhaft, die Nukleinsäure zu modifizieren, um sie gegen den Abbau zu stabilisieren, so dass über einen langen Zeitraum eine hohe Konzentration der Nukleinsäure in der Zelle beibehalten wird (Beigelman et al. (1995) Nucleic Acids Res. 23:3989-94; Dudycz (1995) WO 95/11910; Macadam et al. (1998) WO 98/37240; Reese et al. (1997) WO 97/29116). Typischerweise kann eine solche Stabilisierung durch die Einführung einer oder mehrerer Internukleotid-Phosphorgruppen oder durch die Einführung einer oder mehrerer Nicht-Phosphor-Internukleotide erhalten werden.

Geeignete modifizierte Internukleotide sind in Uhlmann und Peymann (1990 Chem. Rev. 90, 544) zusammengefasst (siehe auch Beigelman et al. (1995) Nucleic Acids Res. 23:3989-94; Dudycz (1995) WO 95/11910; Macadam et al. (1998) WO 98/37240; Reese et al. (1997) WO 97/29116). Modifizierte Intemukleotid-Phosphatreste und/oder Nicht-Phosphorbrücken in einer Nukleinsäure, die bei einer der erfindungsgemäßen Verwendungen eingesetzt werden können, enthalten zum Beispiel Methylphosphonat, Phosphorothioat, Phosphoramidat, Phosphorodithioat und/oder Phosphatester, während Nicht-Phosphor-Internukleotid-Analoge, beispielsweise Siloxanbrücken, Carbonatbrücken, Carboxymethylester, Acetamidatbrücken und/oder Thioetherbrücken enthalten. Es ist auch beabsichtigt, dass diese Modifizierung die Haltbarkeit einer pharmazeutischen Zusammensetzung, die bei einer der erfindungsgemäßen Verwendungen eingesetzt werden kann, verbessert.

Im Rahmen der vorliegenden Erfindung besteht auch die Möglichkeit mit Hilfe des Einsatzes von antisense Oligonukleotiden (Zheng und Kemeny, 1995, Clin. Exp. Immunol. 100:380-2; Nellen und Lichtenstein, 1993, Trends Biochem. Sci. 18:419-23; Stein (1992) Leukemia 6:967-74) und/oder Ribozymen (Amarzguioui, et al. (1998) Cell. Mol. Life Sci. 54:1175-202; Vaish, et al. (1998) Nucleic Acids Res. 26:5237-42; Persidis (1997) Nat. Biotechnol. 15:921-2; Couture und Stinchcomb (1996) Trends Genet. 12:510-5) die Stabilität von Nukleinsäuren zu verringern und/oder die Translation von Nukleinsäuren zu inhibieren. So kann durch die erfindungsgemäße Verwendung der Nukleinsäuresequenzen beispielsweise die Expression der entsprechenden Gene in Zellen sowohl *in vivo* als auch *in vitro* verringert werden. Für die Verwendung als Sonde, oder als "antisense" Oligonukleotid ist eine einzelsträngige DNA oder RNA bevorzugt.

Die Auswirkungen der Modulation eines erfindungsgemäß verwendbaren FGF-BP oder der erfindungsgemäß verwendbaren, identifizierten pharmakologisch aktiven Substanzen können durch verschiedene Testsysteme, die die Aktivität der Zellen und Zellverbände *in vitro* und *in vivo* bestimmen, untersucht werden.

Weiterhin können die das erfindungsgemäße FGF-BP kodierenden Nukleinsäuren in "knock-out" Genkonstrukten oder Expressionskassetten enthalten sein. Solche Knock-out Genkonstrukte sind dem Fachmann zum Beispiel aus den US-Patenten 5,625,122; US 5,698,765; US 5,583,278 und US 5,750,825 bekannt.

Transgene nicht-menschliche Säugetiere, deren Genom mindestens ein erfindungsgemäß verwendbares knock-out Genkonstrukt und/oder mindestens eine erfindungsgemäß verwendbare Expressionskassette wie vorangehend beschrieben enthalten, können zur Diagnose und/oder Prävention und/oder Behandlung von Wundheilungsstörungen, insbesondere diabetes-assoziierte Wunden oder zur Identifizierung von pharmakologisch aktiven Substanzen, verwendet werden. Transgene Tiere, die eine der vorangehend beschriebenen Expressionskassetten enthalten, zeigen in Abhängigkeit des verwendeten Promotors im allgemeinen eine gewebespezifisch erhöhte Expression der Nukleinsäuren und/oder Polypeptide und lassen sich zur Analyse von Wundheilungsstörungen verwenden. So weist beispielsweise eine Activin A transgene Maus eine verbesserte Wundheilung auf (Munz et al. (1999) EMBO J. 18:5205-15) während eine transgene Maus mit dominant negativem KGF Rezeptor eine verzögerte Wundheilung aufweist (Werner et al. (1994) Science 266:819-22). Darüber hinaus können vorangehend beschriebene transgene Tiere mit beschleunigter Wundheilung ausgestattet werden.

Verfahren zur Herstellung von transgenen Tieren, insbesondere von transgenen Mäusen, sind dem Fachmann ebenfalls aus der DE 196 25 049 und den US 4,736,866; US 5,625,122; US 5,698,765; US 5,583,278 und US 5,750,825 bekannt und umfassen transgene Tiere, die beispielsweise über direkte Injektion von Expressionsvektoren (s.o.) in Embryonen oder Spermatozyten, über die Transfektion von Expressionsvektoren in embryonale Stammzellen (Polites und Pinkert: DNA Microinjection and Transgenic Animal Produktion, Seite 15 bis 68 in Pinkert, 1994: Transgenic animal technology: a laboratory handbook, Academic Press, London, UK; Houdebine, 1997, Harwood Academic Publishers, Amsterdam, The Netherlands; Doetschman: Gene Transfer in Embryonic Stem Cells, Seite 115 bis 146 in Pinkert, 1994, supra; Wood: Retrovirus-Mediated Gene Transfer, Seite 147 bis 176 in Pinkert, 1994, supra; Monastersky: Gene Transfere Technology: Alternative Techniques and Applications, Seite 177 bis 220 in Pinkert, 1994, supra) oder über Isolation von Zellkernen aus differenzierten somatische Zellen, beispielsweise Fibroblasten, und anschliessendem Transfer in entkernte Oozyten (Mc Creath et al. (2000) Nature 405:1004-5) erzeugt werden können.

Werden die vorangehend beschriebenen erfindungsgemäß verwendbaren Nukleinsäuren in sogenannte "Targeting" Vektoren oder "knock-out" Genkonstrukte integriert (Pinkert, 1994, supra), können nach Transfektion von embryonalen Stammzellen und homologer Rekombination beispielsweise knock-out Mäuse generiert werden, die im allgemeinen als heterozygote Mäuse verringerte Expression der Nukleinsäure zeigen, während homozygote Mäuse keine Expression der Nukleinsäure mehr aufweisen. Auch die so erzeugten Tiere lassen sich zur Analyse von Wundheilungsstörungen verwenden. So weisen beispielsweise die eNOS- (Lee et al. (1999) Am. J. Physiol. 277:H1600-H1608), Nf-1 (Atit et al. (1999) J. Invest. Dermatol. 112:835-42) und Osteopontin (Liaw et al. (1998) J. Clin. Invest. 101:967-71) knock-out Mäuse eine verschlechterte Wundheilung auf. Auch hier ist eine gewebespezifische Reduktion der Expression wundheilungsrelevanter Gene, beispielsweise in hautspezifischen Zellen unter Verwendung des Cre-loxP Systems (stat3 knock-out, Sano et al. (1999) EMBO J. 18:4657-68), besonders zu bevorzugen. So erzeugte transgene und knock-out Zellen oder Tiere des erfindungsgemäßen FGF-BP Proteins können beispielsweise auch zur Rasteruntersuchung (Screening), zur Identifizierung von pharmakologisch aktiven Substanzen bzw. zum Austesten der Modulationseffizienz gentherapeutisch aktiver Vektoren verwendet werden.

Die mit Hilfe der erfindungsgemäß verwendbaren Testverfahren identifizierten pharmakologisch aktiven Substanzen können, gegebenenfalls kombiniert oder zusammen mit geeigneten Zusatz- und Hilfsstoffen, zur Herstellung eines Diagnostikums oder Arzneimittels zur Diagnose, Prävention und/oder Behandlung von Wundheilungsstörungen, die durch eine verringerte Menge an FGF-BP gekennzeichnet sind, eingesetzt werden, insbesondere bei diabetes-assoziierten, schlecht heilenden Wunden.

Die vorliegende Erfindung betrifft weiterhin die Verwendung mindestens eines erfindungsgemäß verwendbaren FGF-BP Polypeptids gemäß SEQ ID Nr. 1 und 3 und/oder für FGF-BP Polypeptid kodierende Nukleinsäuren gemäß SEQ ID Nr. 2 und 4, oder gegen ein erfindungsgemäß verwendbares FGF-BP Polypeptid gerichteter Antikörper oder Antikörperfragmente und/oder mindestens einer FGF-BP Polypeptid exprimierenden Zelle, gegebenenfalls kombiniert oder zusammen mit geeigneten Zusatz- und Hilfsstoffen, zur Herstellung eines Diagnostikums zur Diagnose von diabetes-assoziierten Wunden.

Beispielsweise kann gemäß der vorliegenden Erfindung anhand einer der beschriebenen Nukleinsäuren ein Diagnostikum auf der Basis der Polymerasekettenreaktion (Beispiele 1 und 2, PCR-Diagnostik, z.B. gemäß EP 0 200 362) oder eines RNase-Protection-Assays (siehe z.B. Sambrook et al., supra Kapitel 7, Seite 7.71-7.78; Werner et al. (1992) Growth Factors and Receptors: A Practical Approach 175-197; Werner (1998) Proc. Natl. Acad. Sci. U.S.A. 89:6896-6900) hergestellt werden. Diese Tests beruhen auf der spezifischen Hybridisierung einer Nukleinsäure mit ihrem komplementären Gegenstrang, üblicherweise der entsprechenden mRNA oder ihrer cDNA. Die erfindungsgemäß verwendbaren Nukleinsäuren können hierbei auch modifiziert sein, wie z.B. in EP 0 063 879 offenbart. . Vorzugsweise wird ein solches DNA-Fragment mittels geeigneter Reagenzien, z.B. radioaktiv mit β-³²P-dCTP oder nicht-radioaktiv mit Biotin oder Digoxigenin, nach allgemein bekannten Methoden markiert und mit isolierter RNA, die vorzugsweise vorher an geeignete Membranen aus z.B. Nitrocellulose oder Nylon gebunden wurde, inkubiert. Bei gleicher Menge an untersuchter RNA aus jeder Gewebeprobe kann somit die Menge an mRNA bestimmt werden, die spezifisch durch die Sonde markiert wurde. Alternativ kann die Bestimmung der mRNA Menge auch direkt in Gewebeschnitten mit Hilfe der *in situ* Hybridisierung (siehe z.B. Werner et al. (1992) Proc. Natl. Acad. Sci. U. S. A. 89:6896-900) erfolgen.

Mit Hilfe eines erfindungsgemäß verwendbaren Diagnostikums kann *in vitro* die Expressionsstärke des jeweiligen Gens in einer Gewebeprobe spezifisch gemessen werden, um beispielsweise eine diabetes-assoziierte Wundheilungsstörung sicher diagnostizieren zu können (Tabelle 3). Insbesondere eignet sich ein solches Verfahren zur frühzeitigen Prognose von Störungen bzw. zur Ermittlung einer Prädispositionen für die Entstehung einer insbesondere diabetes-assoziierten Wunde.

Ein erfindungsgemäß verwendbares Diagnostikum zur Diagnose von diabetes-assoziierten Wundheilungsstörungen umfasst beispielsweise die Verwendung mindestens einer für FGF-BP Polypeptid kodierenden Nukleinsäure gemäß SEQ ID Nr. 2 und 4 hiervon als Sonde, vorzugsweise als DNA-Sonde und/oder als Primer. Eine Sonde im Sinne der vorliegenden Erfindung ist ein definiertes DNA oder RNA Fragment, welches radioaktiv oder chemisch markiert ist und zur Lokalisierung spezifischer Nukleinsäuresequenzen durch Hybridisierung verwendet wird. Ein Primer im Sinne der vorliegenden Erfindung ist eine kurze DNA Sequenz, die eine freie 3' Hydroxylgruppe enthält und an einen Komplementärstrang bindet. Der Primer ist dann Ausgangspunkt für die Kopie des Komplementärstranges. Dies eröffnet eine weitere Möglichkeit, die beschriebenen Nukleinsäuren, zum Beispiel durch die Isolierung aus einer geeignetten Genbank, beispielsweise aus einer wundspezifischen Genbank, anhand einer geeigneten Sonde zu erhalten (siehe z.B. J. Sambrook et al. (1989) Molecular Cloning. A Laboratory Manual 2. Ausgabe, Cold Spring Harbor Laboratory, Cold Spring Harbor, NY Kapitel 8 Seite 8.1 bis 8.81, Kapitel 9 Seite 9.47 bis 9.58 und Kapitel 10 Seite 10.1 bis 10.67).

Als Sonde eignen sich beispielsweise DNA- oder RNA-Fragmente mit einer Länge von ca. 100-1000 Nukleotiden, vorzugsweise mit einer Länge von ca. 200-500 Nukleotiden, insbesondere mit einer Länge von ca. 300-400 Nukleotiden, deren Sequenz gemäß der SEQ ID Nr. 2 und SEQ ID Nr. 4 des Sequenzprotokolls und/oder anhand der cDNA Sequenzen der in Tabelle 1 angegebenen Datenbankeinträge abgeleitet werden kann.

Alternativ können anhand der abgeleiteten Nukleinsäuresequenzen Oligonukleotide synthetisiert werden, die sich als Primer für eine Polymerase Kettenreaktion eignen. Mit diesen können die vorangehend beschriebenen Nukleinsäuren oder Teile dieser aus cDNA, beispielsweise wundspezifischer cDNA, amplifiziert und isoliert werden (Beispiele 1 und 2). Als Primer eignen sich beispielsweise DNA-Fragmente mit einer Länge von ca. 10 bis 100 Nukleotiden, vorzugsweise mit einer Länge von ca. 15 bis 50 Nukleotiden, insbesondere mit einer Länge von 20 bis 30 Nukleotiden deren Sequenz aus den der SEQ ID Nr. 2 und SEQ ID Nr. 4 des Sequenzprotokolls und/oder anhand der cDNA Sequenzen der in der Tabelle 1 angegebenen Datenbankeinträge abgeleitet werden kann.

Eine weitere Ausführungsform der Erfindung betrifft die Verwendung eines Antikörpers oder Antikörperfragments, vorzugsweise eines polyklonalen oder monoklonalen Antikörpers oder Antikörperfragments zur Analyse, Diagnose, von diabetes-assoziierten Wundheilungsstörungen, sowie seine Verwendung zur Identifizierung von pharmakologisch aktiven Substanzen, dadurch gekennzeichnet, dass ein Antikörper produzierender Organismus mit einem erfindungsgemäß verwendbaren Polypeptid immunisiert wird.

Mit Hilfe dieser Antikörper kann beispielsweise eine Gewebeprobe leicht und schnell dahingehend untersucht werden, ob das betreffende Polypeptid in einer verringerten Menge vorhanden ist, um dadurch einen Hinweis auf eine diabetes-assoziierte Wunde, zu erhalten. In diesem Fall sind die erfindungsgemäßen Antikörper beispielsweise mit einem Enzym, wie oben bereits beschrieben, markiert. Der spezifische Antikörper-Peptid-Komplex kann dadurch leicht und ebenso schnell über eine enzymatische Farbreaktion nachgewiesen werden.

Auch kann beispielsweise die lokale Injektion von monoklonalen Antikörpern gegen TGF beta 1 im Tiermodell die Wundheilung verbessern (Ernst et al. (1996) Gut 39:172-5).

Das Verfahren zur Herstellung eines Antikörpers oder Antikörperfragments, vorzugsweise eines polyklonalen oder monoklonalen Antikörpers erfolgt nach dem Fachmann allgemein bekannten Methoden durch Immunisieren eines Säugetiers, beispielsweise eines Kaninchens, mit dem erfindungsgemäßen Polypeptid oder funktioneller Varianten davon, vorzugsweise mit Teilen davon, mit mindestens 6 Aminosäuren Länge, vorzugsweise mit mindestens 8 Aminosäuren Länge, insbesonderte mit mindestens 12 Aminosäuren Länge, gegebenenfalls in Anwesenheit von z.B. Freund's Adjuvans und/oder Aluminiumhydroxidgelen (siehe z.B. Diafond, B. A. et al. (1981) The New England Journal of Medicine:1344-1349). Die im Tier aufgrund einer immunologischen Reaktion entstandenen polyklonalen Antikörper lassen sich anschließend nach allgemein bekannten Methoden leicht aus dem Blut isolieren und z.B. über Säulenchromatographie reinigen. Monoklonale Antikörper können beispielsweise nach der bekannten Methode von Winter & Milstein (Winter, G. & Milstein, C. (1991) Nature, 349, 293-299) hergestellt werden. Als Alternative zu den klassischen Antikörpern können beispielsweise auf Lipocalin basierende sogenannte "Anticaline" verwendet werden (Beste et al. (1999) Proc. Natl. Acad. Sci. USA, 96:1898-1903). Die natürlichen Ligandenbindenden "Sites" der Lipocaline, wie z.B. das Retinol-bindende Protein oder das Bilin-bindende Protein können beispielsweise durch einen "kombinatorischen Protein Design" Ansatz in einer Weise verändert werden, dass sie an ausgewählte Haptene, beispielsweise an die erfindungsgemäß verwendbaren Polypeptide binden (Skerra, 2000, Biochim. Biophys. Acta 1482:337-50). Weitere bekannte "scaffolds" als Alternativen für Antikörper für die molekulare Erkennung sind bekannt (Skerra, J. Mol. Recognit., 2000, 13:167-187).

Der erfindungsgemäß verwendbare Antikörper oder das Antikörperfragment sind gegen ein erfindungsgemäßes Polypeptid gerichtet ist und reagieren spezifisch mit den erfindungsgemäßen Polypeptiden, wobei die oben genannten Teile des Polypeptids entweder selbst immunogen sind oder durch Kopplung an geeignete Träger, wie z.B. bovines Serumalbumin, immunogen gemacht bzw. in ihrer Immunogenität gesteigert werden können. Dieser erfindungsgemäß verwendbare Antikörper ist entweder polyklonal oder monoklonal, bevorzugt ist ein monoklonaler Antikörper. Unter dem Begriff Antikörper oder Antikörperfragment versteht man gemäß der vorliegenden Erfindung auch gentechnisch hergestellte und gegebenenfalls modifizierte Antikörper bzw. antigenbindende Teile davon, wie z.B. chimäre Antikörper, humanisierte Antikörper, multifunktionelle Antikörper, bi- oder oligospezifische Antikörper, einzelsträngige Antikörper, F(ab)- oder F(ab)₂-Fragmente (siehe z.B. EP-B1-0 368 684, US 4,816,567, US 4,816,397, WO 88/01649, WO 93/06213, WO 98/24884). So wurden Antikörper gegen das erfindungsgemäße FGF-BP bereits erfolgreich hergestellt und verwendet (Liu et al. (2001) Kidney Int., 59:1717-1728; Sauter et al. (2001) Int. J. Cancer 92:374-381).

Um pharmakologisch aktive Substanzen als Diagnostikum zu verwenden, können die Substanzen einen detektierbaren Marker enthalten, beispielsweise kann die Substanz radioaktiv markiert, Fluoreszenz-markiert oder Lumineszenz-markiert sein. Weiterhin können Substanzen an Enzyme gekoppelt werden, die eine indirekte Detektion erlauben, beispielsweise wie oben beschrieben über enzymatische Katalyse mittels eines Peroxidase-Assays mit einem chromogenen Substrat oder durch Bindung eines markierten oder detektierbaren Antikörpers. Die Substanzen können dann mit einer zu untersuchenden Probe in Kontakt gebracht werden und so die Menge eines erfindungsgemäß verwendbaren Polypeptids oder einer funktionellen Variante davon oder dieses kodierende Nukleinsäure oder einer Variante davon, oder einer ein erfindungsgemäß verwendbares Polypeptid oder einer funktionellen Variante davon oder einer dieses kodierende Nukleinsäure oder eine Variante davon exprimierenden Zelle oder eines gegen ein erfindungsgemäß vierwendbares Polypeptid gerichteten Antikörpers oder eines Antikörperfragments in der Probe bestimmt werden. Die Ergebnisse dieser Probe, die einem zu untersuchenden Organismus entstammt, kann dann verglichen werden mit dem Ergebnis einer Probe, die einem gesunden oder kranken Organismus entstammt.

Die Erfindung beschreibt ferner die Verwendung mindestens eines erfindungsgemäß verwendbaren Polypeptids oder dieses kodierende Nukleinsäure oder eines gegen ein erfindungsgemäß verwendbares Polypeptid gerichteten Antikörpers oder eines Antikörperfragments gegebenenfalls kombiniert oder zusammen mit geeigneten Zusatz- und Hilfsstoffen, zur Herstellung eines auf einem Trägermaterial fixierten Arrays zur Analyse in Zusammenhang mit diabetes-assoziierten, schlecht heilende Wunden.

Verfahren zur Herstellung von solchen Arrays mittels Festphasenchemie und photolabilen Schutzgruppen sind zum Beispiel aus der US 5,744,305 bekannt.

Zur Analyse in Zusammenhang mit diabetes-assoziierten Wunden, können beispielsweise auch DNA-Chips und/oder Protein-Chips, die mindestens eine Nukleinsäure, mindestens ein Polypeptid und/oder mindestens einen Antikörper oder Antikörperfragment, wie vorangehend beschrieben, umfassen. DNA-Chips sind zum Beispiel aus der US 5,837,832 bekannt.

Die Erfindung soll nun im folgenden anhand der Tabellen und Beispiele weiter verdeutlicht werden, ohne dass die Erfindung hierauf eingeschränkt wird.

**Tabellen**

| | |
|---|---|
| **Tabelle 1** | zeigt die Auflistung der erfindungsgemäß verwendbaren FGF-BP Polypeptide und Nukleinsäuren. |
| **Tabelle 2** | zeigt die mittels "Taq-Man"-Analyse bestimmte Kinetik der differenziellen Regulation der FGF-BP Expression bei der Wundheilung normaler und diabetischer Mäuse als Beispiel für eine Wundheilungsstörung, die mit einem Mangel an FGF-BP im Zusammenhang steht. |
| **Tabelle 3** | zeigt die mittels "Taq-Man"-Analyse bestimmte differenzielle Regulation der FGF-BP Expression in drei verschiedenen Wundheilungszuständen des Menschen im Vergleich (normale Wundheilung, venöser und diabetischer Ulkus) als Beispiel für die selektiv bei diabetischen Patienten auftretende Prädisposition für eine Wundheilungsstörung, die durch einen Mangel an FGF-BP verursacht wird. |

Die Polypeptidsequenzen des murinen und humanen FGF-BP, sowie deren kodierende Nukleotidsequenzen sind gemäß SEQ ID Nr. 1 bis SEQ ID Nr. 4 aufgeführt.

Die für die Beispiele verwendeten Oligonukleotide sind gemäß SEQ ID Nr. 5 bis SEQ ID Nr. 12 aufgeführt.

### Beispiele

### Beispiel 1: Differenzielle Expression der FGF-BP mRNA in humanen Biopsien aus gesunder Haut und Wunden von gesunden Probanden, sowie Biopsien venöser und diabetischer Ulzera

In einem ersten Experiment sollte untersucht werden, inwieweit die Expression von FGF-BP in verschiedenen Wundheilungserkrankungen reguliert ist. Dazu wurden von 6 gesunden Probanden aus unbehandelter intakter Haut, aus Wunden 1 Stunde nach Wundsetzung, aus Tag 1- Wunden, sowie aus Tag 5- und Tag 15-Wunden mittels 4 mm bzw. 6 mm Stanzen Hautproben entnommen und Biopsien jedes Zeitpunktes gepoolt. Außerdem wurde von 6 Patienten mit chronischen venösen Ulzera, sowie von 4 Patienten mit diabetischen Ulzera Stanzbiopsien gleichzeitig sowohl von intakter Haut als auch vom Wundgrund und Wundrand entnommen und anschließend jede Gruppe (intakte Haut, Wundgrund, Wundrand) für sich gepoolt.

Aus allen Biopsien wurde RNA isoliert. Dazu wurden die Biopsien in RNAclean Puffer (AGS, Heidelberg), dem 1/100 Volumenanteil 2-Mercaptoethanol zugesetzt worden war, unter Verwendung eines Dispersers homogenisiert. Anschließend wurde die RNA durch zweimaliges Phenolisieren mittels mit Wasser gesättigtem saurem Phenol und in Gegenwart von 1-Bromo-3-Chloro-Propan extrahiert. Anschließend wurden eine Isopropanol- und eine Ethanol-Fällung durchgeführt und die RNA mit 75% Ethanol gewaschen. Danach wurde ein DNase I Verdau der RNA durchgeführt. Hierzu wurden 20 µg RNA (ad 50 µl mit DEPC-behandeltem Wasser) mit 5,7 µl Transkriptions-Puffer (Roche), 1 µl RNase Inhibitor (Roche; 40 U/µl) und 1 µl DNase I (Roche; 10 U/µl) 20 min bei 37°C inkubiert. Dann wurde wiederum 1 µl DNase I zugegeben und weitere 20 min bei 37°C inkubiert. Anschließend wurde die RNA phenolisiert, Ethanol-gefällt und gewaschen. Alle oben aufgeführten Schritte wurden mit DEPC (Diethylpyrocarbonat)-behandelten Lösungen bzw. Flüssigkeiten, durchgeführt soweit diese keine reaktiven Aminogruppen enthielten.

Anschließend erfolgte die Herstellung von cDNA aus der extrahierten RNA. Dies erfolgte in Gegenwart von 1 x TaqMan RT-Puffer (Perkin Elmer), 5,5 mM MgCl₂ (Perkin Elmer), je 500 µM dNTPs (Perkin Elmer), 2,5 µM Random Hexamere (Perkin Elmer), 1,25 U/µl MultiScribe Reverse Transcriptase (50 U/µl Perkin Elmer), 0,4 U/µl RNase Inhibitor (20 U/µl, Perkin Elmer), 20 µl RNA (50 ng/µl) und DEPC-behandeltem Wasser (ad 100 µl Volumen). Nach Zugabe der RNA und guter Durchmischung wurde die Lösung auf zwei 0,2 ml Gefäße verteilt (je 50 µl) und die reverse Transkription in einem Temperatur-Cycler durchgeführt (10 min bei 25°C; 30 min bei 48°C und 5 min bei 95°C). Die nachfolgende Quantifizierung der cDNA erfolgte mittels quantitativer PCR unter Verwendung des SYBR Green PCR Master Mixes (Perkin Elmer), wobei für die Bestimmung der FGF-BP cDNA Menge eine Dreifachbestimmung durchgeführt wurde. Dazu wurden anhand der FGF-BP kodierenden SEQ ID Nr. 1 Primer gemäß der SEQ ID Nr. 5 und SEQ ID Nr. 6 ausgewählt. Als Referenz wurde Cyclophilin A (Gen Bank: XM039526) verwendet, welches gemäß den Primern der Sequenz unter SEQ ID Nr. 7 und SEQ ID Nr. 8 amplifiziert wurde. Die Stammlösung für jedes Triplett enthielt bei 57 µl Gesamtvolumen 37,5 µl 2 x SYBR Master Mix, 0,75 µl AmpErase UNG (1 U/µl) und 18,75 µl DEPC-behandeltes Wasser. Pro Dreifachbestimmung wurden zu 57 µl Stammlösung je 1,5 µl Vorwärts- und Rüchwärts-Primer (gemäß SEQ ID Nr. 5 und SEQ ID Nr. 6) in einem zuvor optimierten Konzentrationsverhältnis hinzugefügt. Je 60 µl der Stammlösung/Primer-Mischung wurde mit 15 µl cDNA-Lösung (2 ng/µl) gemischt und auf drei wells verteilt. Parallel dazu wurde eine Stammlösung mit Primern zur Bestimmung von Cyclophilin A (SEQ ID Nr. 7 und SEQ ID Nr. 8) hergestellt, mit weiteren 15 µl der gleichen cDNA-Lösung gemischt und auf drei wells verteilt.

Außerdem wurden, um eine Standardkurve für die Cyclophilin-PCR zu erstellen, verschiedene cDNA-Lösungen als Verdünnungsreihe hergestellt (4 ng/µl; 2 ng/µl; 1 ng/µl; 0,5 ng/µl und 0,25 ng/µl). Je 15 µl dieser cDNA-Lösungen wurden mit 60 µl Stammlösung/Primer-Mischung zur Bestimmung von Cyclophilin gemischt und auf drei wells verteilt. Ebenso wurde eine Standardkurve für die humane FGF-BP-PCR erstellt; dabei wurden dieselben Verdünnungen, die auch für die Cyclophilin-Standardkurve zum Einsatz kamen, verwendet. Als Kontrolle diente ein PCR Ansatz ohne cDNA. Zu jeweils 60 µl Stammlösung/Primer-Mischung von jeweils humanem FGF-BP und Cyclophilin wurden je 15 µl DEPC-Wasser gegeben, gemischt und jeweils auf drei wells verteilt. Die Amplifikation der Ansätze wurde im GeneAmp 5700 durchgeführt (2 min bei 50°C; 10 min bei 95°C, gefolgt von 3 Zyklen mit 15 sek bei 96°C und 2 min bei 60°C; danach 37 Zyklen mit 15 sek bei 95°C und 1 min bei 60°C). Die Auswertung erfolgte durch die Bestimmung der relativen Abundanz von FGF-BP in Bezug auf die Cyclophilin Referenz. Dazu wurde zuerst eine Standardkurve erstellt, indem die C_{T}-Werte der Verdünnungsreihe gegen den Logarithmus der cDNA-Menge im PCR-Ansatz (in ng umgeschriebener RNA) aufgetragen wurden und die Steigung (s) der Geraden ermittelt wurde. Die Effizienz (E) der PCR ergibt sich dann wie folgt: E = 10^{-l/s}-1. Die relative Abundanz (X) der untersuchten cDNA Spezies FGF-BP (Y) in Bezug auf Cyclophilin ist dann: X=(1+E_{Cyclophilin})^{C}_{T}^{(Cyclophilin)}/(1+E_{Y})^{C}_{T}^{(Y)}. Anschließend wurden die Zahlenwerte normiert, indem die Menge an cDNA aus intakter Haut gesunder Probanden 1 gesetzt wurden.

Die relativen Änderungen der FGF-BP Expression in verschiedenen Wundheilungszuständen sind in Tabelle 3 zusammengestellt. Es zeigt sich, dass in allen Wundheilungszuständen gesunder Probanden eine 2- bis 3- fach erhöhte FGF-BP Expression im Vergleich zu intakter Haut zu beobachten ist, während Patienten, die unter venösen, schlecht heilenden Ulzera leiden, eine generell erhöhte Expression an FGF-BP aufweisen. Im Gegensatz dazu exprimieren Patienten, die unter diabetischen, schlecht heilenden Ulzera leiden, generell zu wenig FGF-BP. Daraus ergibt sich, dass die Ursache für die schlechte Wundheilung diabetischer Patienten in einer generellen, jedoch für diese Erkrankung gegenüber beispielsweise venösen Wunden selektiven, verminderten Abundanz des FGF-BP begründet ist. Somit sind diabetische Patienten gegenüber gesunden Probanden und Patienten, die an venösen Ulzera leiden, durch einen Mangel an FGF-BP für eine schlechtere Wundheilung prädisponiert. Die Ergebnisse dieses Experiments zeigen, dass eine erfolgsversprechende Therapie der Wundheilungsstörung, insbesondere diabetes-assoziierter Wunden, darin bestehen kann, die Menge und/oder Aktivität der FGF-BP Polypeptide und/oder Nukleinsäuren lokal zu erhöhen und damit eine verbesserte Wundheilung zu erreichen.

### Beispiel 2: Differenzielle Expression von FGF-BP in murinen Wunden

Um zu bestätigen, dass FGF-BP tatsächlich besonders zur Behandlung, Diagnose und/oder Prävention von Wundheilungsstörungen, die durch einen Mangel an FGF-BP kennzeichnet sind, insbesondere von diabetes-assoziierten schlecht heilenden Wunden geeignet ist, wurde der Einfluss von FGF-BP auf die Wundheilung in Tiermodellen untersucht. Die Bestätigung der humanen Daten in Tiermodellen ist eine Voraussetzung, um geeignete Zeitpunkte für die Behandlung, die Diagnose und/oder Prävention von Wundheilungsstörungen, die durch einen Mangel an FGF-BP gekennzeichnet sind, insbesondere von diabetes-assoziierten Wunden, zu ermitteln. Da Mäuse sich als geeignetes Modellsystem für die Untersuchung der Wundheilung im Menschen erwiesen haben, wurde die Expression des FGF-BP in murinen Biopsien von verschiedenen Zeitpunkten der Wundheilung bestimmt. Dabei wurden zum Zeitpunkt 1h, 7h, 15h, 24h, 3 Tage, 5 Tage, 7 Tage sowie 14 Tage nach Wundsetzung Stanzbiopsien aus Wunden und intakter Haut aus normal heilenden Kontrollmäusen (C57BL/6) und diabetischen Mäusen (C57BL/Ks-db/db/Ola) gewonnen.

Die Biopsien wurden homogenisiert und RNA isoliert, wie in Beispiel 1 beschrieben. Danach erfolgte ein DNase Verdau und das Umschreiben in cDNA. Die Quantifizierung wundheilungsrelevanter cDNAs erfolgte ebenfalls wie in Beispiel 1 beschrieben. Für die spezifische Amplifikation von murinem FGF-BP wurden anhand der Sequenz des FGF-BP (SEQ ID Nr. 4) Primer mit der Sequenz der SEQ ID Nr. 9 und SEQ ID Nr. 10 ausgewählt. Als Referenzgen wurde in diesem Experiment GAPDH benutzt. Dazu wurden für die PCR-Amplifikation anhand der Sequenz des GAPDH Genes (Gen Bank: M17851) Primer mit der Sequenz der SEQ ID Nr. 11 und SEQ ID Nr. 12 verwendet. Für die Quantifizierung wurden je Ansatz cDNA aus 10 ng revers transkribierter Gesamt-RNA in einem Gesamtvolumen von 25 µl amplifiziert. Die PCR wurde entsprechend den Angaben des Herstellers (PE Applied Biosystems, SYBR Green PCR and RT-PCR Reagents Protocol, 1998) durchgeführt. Die C_{T}-Werte wurden analysiert und daraus wurde die Häufigkeit von FGF-BP mRNA relativ zu GAPDH mRNA berechnet. Die Ergebnisse der Experimente sind in Tabelle 2 zusammengestellt und zeigen, dass während des gesamten Beobachtungszeitraums bei diabetischen Tieren FGF-BP mRNA während der Wundheilung relativ zu intakter Haut deutlich vermindert (im Schnitt um Faktor 2,8) exprimiert ist. Werden diese Ergebnisse mit den Daten der Wundheilungskinetik normaler Mäuse verglichen, zeigt sich, dass sich bei normalen Mäusen während der Wundheilung die relative Expression der FGF-BP mRNA während der Wundheilung praktisch nicht ändert. Diese Resultate verdeutlichen, dass sich die im Menschen beobachtete selektiv in diabetischen Patienten verminderte Expression des FGF-BP als Ursache für die Wundheilungsstörung dieser Patientengruppe auch auf die Maus übertragen lässt. Somit dient die diabetische Maus als Modell für die Etablierung therapierelevanter Daten, wie zum Beispiel geeignete Zeitpunkte und Darreichungsform.

### Beispiel 3: Behandlung muriner Wunden mit FGF-BP durch gentherapeutische Methoden

Um zu bestätigen, dass FGF-BP tatsächlich besonders zur Behandlung und/oder Prävention diabetes-assoziierter schlecht heilender Wunden geeignet ist, wird der Einfluss von FGF-BP auf die Wundheilung *in vivo* getestet. Dazu wird die Wundheilung nach Applikation der FGF-BP cDNA gemäß SEQ ID Nr. 4 in männlichen diabetischen Sprague Dawley Ratten verfolgt. Zur Quantifizierung der Wundheilung wird die Zerreißfestigkeit (tensile strength) der Wunden untersucht, wobei eine höhere Zerreißfestigkeit eine verbesserte Wundheilung widerspiegelt. Das Tiermodell der diabetischen Ratte ist ein etabliertes Modellsystem zur Untersuchung diabetes-assoziierter schlecht heilender Wunden (Davidson, Arch. Dermatol. Res. (1998) 290: S1-S11).

Zunächst wird für dieses Experiment ein geeigneter Expressionsvektor beispielsweise auf der Grundlage des Vektors pMH (F. Hoffman-La Roche) konstruiert, indem zwischen dem CMV Promotor und der Multiple Cloning Site in die HindIII Schnittstelle das Intron II des Insulingens aus der Ratte eingefügt wird (pMHInt). Unter Verwendung der Multiple Cloning Site wird dann die erfindungsgemäße FGF-BP cDNA in den modifizierten Expressionsvektor pMHInt kloniert. Hierzu wird der kodierende Bereich der FGF-BP cDNA gemäß SEQ ID Nr. 4 mittels geeigneter PCR amplifiziert und anschließend mit Restriktionsenzymen geschnitten, die eine "in-frame" Ligation der cDNA in den mit den gleichen Restriktionsenzymen geschnittenen, modifizierten Expressionsvektor erlauben. Somit wird ein Expressionplasmid pMHInt_FGF-BP erhalten. Als Kontrollvektor wird pMHInt verwendet, der beispielsweise ein Luziferase-Gen enthält (pMHIntLuc).

Zur Induktion der Diabetes wird vier Ratten mit einem Körpergewicht von 250-300g eine frisch hergestellte wässrige Streptozotocin-Lösung (Sigma) i.p. injiziert (50 mg/kg Körpergewicht). 7-9 Tage nach Induktion wird der Blutzucker der Tiere überprüft. Ein Blutzuckerspiegelwert von über 200 mg/dL dient als unterer Grenzwert für die Beurteilung des diabetischen Zustands der Tiere.

Die vier diabetischen Ratten sowie die vier nicht-diabetischen Kontrolltiere werden anschließend mit einer Mischung aus 2% O₂ (2 l/min) und 1,25% Isofluran betäubt. Der Rücken wird enthaart und an vier Stellen pro Tier wird auf dem Rükken für die spätere Wundsetzung eine Marke gesetzt. Diese Marken werden beispielsweise mit jeweils 0,5 µg Plasmid DNA, die an Goldpartikeln (BioRad) gebunden ist, unter Verwendung der "Helios Gene Gun" (BioRad) mit 500 psi beschossen, wobei jeweils zwei Stellen mit dem FGF-BP-Expressionsvektor pMHInt_FGF-BP und zwei Stellen mit dem Kontrollvektor pMHIntLuc beschossen werden. Dabei erfolgt jeweils ein Beschuss mit pMHInt_FGF-BP am anterioren und ein Beschuss am posterioren Ende der Marke. Anschließend werden 1 cm lange Schnittwunden durch die beschossenen Stellen hindurch gesetzt und die Wunden mit Wundklammern verschlossen. Nach 10 Tagen wird die Zerreißfestigkeit der Wunden unter Verwendung eines Tensiometers (BTC-2000, SRLI, Nashville) nach den Angaben des Herstellers bestimmt. Anschließend wird der Quotient (E/C-Wert) gebildet aus dem absoluten Wert der Zerreißfestigkeit einer mit pMHInt_FGF-BP beschossenen Wunde und dem absoluten Wert der Zerreißfestigkeit einer mit dem Kontrollvektor pMHIntLuc beschossenen Wunde desselben Tieres. Die Bestimmung des Mittelwertes der E/C-Werte erlaubt die Ermittlung der Änderungen der Zerreißfestigkeit in Abhängigkeit von erfindungsgemäßem FGF-BP zwischen diabetischen und Kontrolltieren. Dabei deutet eine erhöhte Zerreißfestigkeit von pMHInt_FGF-BP behandelten Wunden in diabetischen Tieren auf eine Eignung von FGF-BP zur selektiven Behandlung von diabetes-assoziierten, schlecht heilenden Wunden.

Alternativ können natürlich auch andere Expressionsvektoren sowie andere Applikationsformen der Nukleinsäuren gewählt werden. So können Nukleinsäuren beispielsweise mit Liposomen komplexiert werden, um ein Eindringen in die Zelle zu erleichtern oder die Nukleinsäure kann an eine Trägermatrix, beispielsweise Kollagen, gebunden werden, die dann auf die Wunde appliziert wird. Ein Eindringen der Nukleinsäure in die Hautzellen ist dabei möglich. Um die Schwierigkeit des Eindringens in die Zelle zu überwinden können auch virale Vektoren, beispielsweise Adeno- oder Retroviren, verwendet werden, die dann über Infektion Zugang zur Zellen erhalten.

Ebenso können beispielsweise auch humanes FGF-BP Polypetid kodierende Nukleinsäuren gemäß SEQ ID Nr. 2 oder Varianten davon verwendet werden.

### Beispiel 4: Behandlung muriner Wunden durch in vivo Applikation von FGF-BP Polypeptiden

Da das erfindungsgemäß verwendbare FGF-BP Polypeptid einen extrazellulären Wirkungsort besitzt und somit ein Eindringen in die Zellen nicht notwendig ist, wird die Eignung topisch administrierter, rekombinant hergestellter FGF-BP Polypeptide zur Behandlung und/oder Prävention diabetes-assoziierter schlecht heilender Wunden für die Wundheilung *in vivo* getestet.

Zunächst werden beispielsweise mit Hilfe des Baculovirus-Expressionssystems (BAC-TO-BAC Baculovirus Expression System, Life Technologies INC., Gaithersburg, MD) FGF-BP Polypeptide hergestellt. Dazu wird zunächst die cDNA des erfindungsgemäß verwendbaren FGF-BP gemäß SEQ ID Nr. 4 via PCR amplifiziert (siehe Experiment 3) und beidseits mit je einem (His)6-Tag versehen. Diese modifizierte cDNA wird in geeignete Restriktionsschnittstellen des pFASTBAC HTb Donor Plasmids kloniert. Anschließend wird das Plasmid in den Bacmid-enthaltenden DH10BAC *E.coli*-Stamm transformiert. Isolierte, rekombinante Bacmid-DNA wird dann in Sf-9 Zellen transfektiert, um entsprechende Baculoviren zu generieren. Nach fünf Tagen werden die Zellen geerntet, pelletiert und mit Hilfe von 6 M Guanidinium_HC1, 0,01 M HCl, 0,1 M Natriumphosphat pH 8,0 lysiert. Die Zelllysate werden eine Stunde auf Eis inkubiert, zelluläre Überreste abzentrifugiert und der Überstand auf eine Nickel-NTA Sepharose Säule (Qiagen, Hilden, Germany) geladen. Die Säule wird wiederholt mit 30 mM Natrium Citrat, 300 mM Natrium Chlorid und absteigenden pH Werten von 8, 6,3, 5,9 und 5,7 gewaschen. His-getagtes FGF-BP wird dann mit 3x0,5 ml Puffer mit pH 4,5 eluiert und neutralisiert. Auf diese Art hergestelltes, rekombinantes FGF-BP Polypeptid wird anschließend in verschiedenen Konzentrationen beispielsweise in einem inerten Hydrogel (2,3% modifizierte Methylzellulose, 20% Propylen_Glykol und 77,7% Wasser) formuliert (Intra Site von Smith & Nephew).

Zur Induktion von Diabetes werden vier Ratten, wie in Experiment 3 beschrieben, mit Streptozotocin-Lösung behandelt. Vier dieser diabetischen Ratten sowie vier nicht-diabetische Kontrolltieren werden anschließend wie bereits beschrieben betäubt und enthaart. In die epilierte Rückenhaut eines Tieres werden dann mit Hilfe einer Biopsiestanze vier Wunden gestanzt. Auf zwei dieser Wunden werden in einem Gesamtvolumen von 50 µl pro Wunde die formulierten FGF-BP Polypeptide, auf die verbleibenden zwei Wunden als Kontrolle lediglich die Trägersubstanz ohne FGF-BP topisch auf den Wundrand aufgetragen. Die Wunden werden einzeln mit einem semi-okklusiven Verband (OpSite) versorgt. Die Progression der Reepithelialisierung der Wunde wird photopraphisch während 10 Tagen dokumentiert (EPICAM) und mit dem Wert bei Tag 0 (sofort nach Verwundung) verglichen. Eine beschleunigte Reepithelialisierungsrate nach Applikation von FGF-BP Polypeptiden selektiv bei diabetischen Tieren deutet auf eine besondere Eignung der FGF-BP Polypeptide zur selektiven Behandlung von diabetes-assoziierten, schlecht heilenden Wunden hin.

Alternativ können auch andere Expressionssysteme, die die Herstellung eines korrekt gefalteten, biologisch aktiven FGF-BP Polypeptids erlauben, sowie andere Formulierungen oder auch die intradermale Injektion von Proteinen in geeigneten Trägerlösungen verwendet werden. Neben dem murinen Protein können auch humane FGF-BP Polypeptide gemäß SEQ ID Nr. 1 oder funktionelle Varianten davon eingesetzt werden.

Somit kann nachgewiesen werden, dass FGF-BP wirksam bei der Prävention und/oder Behandlung von diabetes-assoziierten, schlecht heilenden Wunden ist. Die Wirkung von FGF-BP ist besonderes geeignet für diese diabetes-assoziierten Wundheilungsstörungen im Vergleich zur Behandlung anderer Wundheilungsstörungen, da bei letzteren keine Fehlregulation der FGF-BP Expression vorliegt.

Zur Prävention und/oder Behandlung ist die Menge an FGF-BP, vorzugsweise an funktionell aktivem FGF-BP im Wundbereich zu erhöhen. Eine bevorzugt zu behandelnde Indikation ist der diabetische Ulkus. Die Administration eines Medikaments enthaltend FGF-BP erfolgt vorzugsweise topisch, insbesondere mittels Gentherapie (Beispiel 3). Die Administration kann weiterhin besonders bevorzugt durch die topische Applikation eines rekombinanten, verwendungsgemäßen FGF-BP-Polypeptids erfolgen (Beispiel 4), da der Wirkort des FGF-BP Polypeptids extrazellulär ist und somit das Eindringen des Proteins in Zellen nicht nötig ist.

**Tabelle 1**

| **Name** | **Organismus** | **Protein** | **SEQ ID No.** | **cDNA** | **SEQ ID No.** |
|---|---|---|---|---|---|
| humanes FGF-BP | *Homo sapiens* | TrEMBL: Q14512 | 1 | GenBank: M60047 | 2 |
| murines FGF-BP | *Mus musculus* | TrEMBL: 070514 | 3 | GenBank: AF065441 | 4 |

**Tabelle 2**

| **Zeitpunkt nach Wundsetzung** | **Rel. mFGF-BP cDNA Menge in gesunden Mäusen** | **Rel. mFGF-BP cDNA in diabetischen Mäusen** |
|---|---|---|
| Intakte Haut | 1.0 | 1.0 |
| 1 d | 1.0 | 0.3 |
| 3 d | 1.0 | 0.5 |
| 5 d | 0.9 | 0.5 |
| 7 d | 1.1 | 0.3 |
| 14 d | 1.5 | 0.4 |

**Tabelle 3**

| **Biopsie** | **Rel. FGF-BP cDNA Menge** |
|---|---|
| Patienten-Pool 1: Intakte Haut Gesunde Patienten | 1.00 |
| Patienten-Pool 2 Stunde 1-Wunde Gesunde Patienten | 3.10 |
| Patienten-Pool 3 Tag 1-Wunde Gesunde Patienten | 2.70 |
| Patienten-Pool 4 Tag 5-Wunde Gesunde Patienten | 1.80 |
| Patienten-Pool 5 Tag 15-Wunde Gesunde Patienten | 1.10 |
| Patienten Pool 6 Intakte Haut Ulkus Patienten | 1.60 |
| Patienten Pool 7 Wundrand Ulkus Patienten | 1.50 |
| Patienten Pool 8 Wundgrund Ulkus Patienten | 0.01 |
| Patienten Pool 9 Intakte Haut Patienten mit diabetischem Ulkus | 0.50 |
| Patienten Pool 10 Wundrand Patienten mit diabetischem Ulkus | 0.60 |
| Patienten Pool 11 Wundgrund Patienten mit diabetischem Ulkus | 0.00 |

## Patentansprüche

1. Verwendung mindestens eines FGF-BP Polypeptids gemäß SEQ ID Nr. 1 und SEQ ID Nr. 3 oder mindestens einer für FGF-BP kodierenden Nukleinsäure gemäß SEQ ID Nr. 2 und SEQ ID Nr. 4 oder gegen ein FGF-BP Polypeptid gemäß SEQ ID Nr. 1 oder SEQ ID Nr. 3 gerichteter Antikörper oder Antikörperfragmente und/oder einer ein FGF-BP Polypeptid gemäß SEQ ID Nr. 1 oder SEQ ID Nr. 3 exprimierenden Zelle zur Herstellung eines Arzneimittels zur Behandlung, Diagnose und/oder Prävention von diabetes-assoziierten Wundheilungsstörungen.

2. Verwendung nach Anspruch 1, **dadurch gekennzeichnet, dass** das Arzneimittel geeignete Zusatz- und/oder Hilfsstoffe aufweist.

3. Verwendung nach Anspruch 1 oder 2, **dadurch gekennzeichnet, dass** die Nukleinsäure nach SEQ ID Nr. 2 und 4 in Form eines Expressionsvektors, insbesondere eines gentherapeutisch wirksamen Vektors eingesetzt wird.

4. Verwendung nach Anspruch 1 oder 2, **dadurch gekennzeichnet, dass** es sich bei den genannten Zellen um humane, nicht-embryonale, autologe oder allogene Zellen handelt.

5. Verwendung nach Anspruch 4, **dadurch gekennzeichnet, dass** die Zellen Hautzellen, insbesondere Keratinozyten, Fibroblasten oder Endothelzellen sind.

6. Verwendung mindestens eines FGF-BP Polypeptids gemäß SEQ ID Nr. 1 und 3 und/oder mindestens einer für FGF-BP kodierenden Nukleinsäure gemäß SEQ ID Nr. 2 und 4 oder gegen ein FGF-BP Polypeptid gemäß SEQ ID Nr. 1 oder SEQ ID Nr. 3 gerichteter Antikörper oder Antikörperfragmente und/oder mindestens einer FGF-BP Polypeptid gemäß SEQ ID Nr. 1 oder SEQ ID Nr. 3 exprimierenden Zelle zur *in vitro* Identifizierung pharmakologisch aktiver Substanzen, die die Funktion und/oder die Expression des FGF-BP Polypeptids und/oder einer für FGF-BP Polypeptid kodierenden Nukleinsäure steigern zur Behandlung und/oder Prävention von diabetes-assoziierten Wunden.

7. Verwendung mindestens eines FGF-BP Polypeptids gemäß SEQ ID Nr. 1 und 3 und/oder mindestens einer für FGF-BP kodierenden Nukleinsäure gemäß SEQ ID Nr. 2 und 4 oder gegen ein FGF-BP Polypeptid gemäß SEQ ID Nr. 1 oder SEQ ID Nr. 3 gerichteter Antikörper oder Antikörperfragmente und/oder mindestens einer FGF-BP Polypeptid gemäß SEQ ID Nr. 1 oder SEQ ID Nr. 3 exprimierenden Zelle zur *in vitro* Identifizierung pharmakologisch aktiver Substanzen, die die Funktion und/oder die Expression des FGF-BP Polypeptids und/oder einer für FGF-BP Polypeptid kodierenden Nukleinsäure steigern zur Behandlung und/oder Prävention von diabetischen Wundheilungsstörungen.

8. Verwendung mindestens eines FGF-BP Polypeptids gemäß SEQ ID Nr. 1 und 3 und/oder mindestens einer für FGF-BP kodierenden Nukleinsäure gemäß SEQ ID Nr. 2 und 4 oder gegen ein FGF-BP Polypeptid gemäß SEQ ID Nr. 1 oder SEQ ID Nr. 3 gerichteter Antikörper oder Antikörperfragmente und/oder mindestens einer FGF-BP Polypeptid gemäß SEQ ID Nr. 1 oder SEQ ID Nr. 3 exprimierenden Zelle zur Herstellung eines Tests zur Auffindung von pharmakologisch aktiven Substanzen in Zusammenhang mit diabetes-assoziierten Wunden.

9. Verwendung nach Anspruch 8, **dadurch gekennzeichnet, dass** der Test geeignete Zusatz- und/oder Hilfsstoffe aufweist.

10. Verwendung nach mindestens einem der Ansprüche 1, 6, 7, 8 und 9, **dadurch gekennzeichnet, dass** die Nukleinsäure eine DNA oder RNA, vorzugsweise eine DNA, besonders bevorzugt eine doppelsträngige DNA ist.

11. Verwendung nach mindestens einem der Ansprüche 6, 7, 8 und 9, **dadurch gekennzeichnet, dass** mindestens ein FGF-BP Polypeptid oder diese kodierende Nukleinsäuren oder gegen FGF-BP Polypeptide gerichtete Antikörper oder Antikörperfragmente an eine Festphase gebunden sind, und mindestens eine Substanz auf ihre pharmakologische Aktivität hin untersucht wird.

12. Verwendung nach mindestens einem der Ansprüche 6, 7, 8 und 9, **dadurch gekennzeichnet, dass** mindestens ein FGF-BP Polypeptid von mindestens einer Zelle exprimiert wird und mindestens eine Substanz auf ihre pharmakologische Aktivität hin untersucht wird.

13. Verwendung nach mindestens einem der Ansprüche 1, 6, 7, 8 und 9, **dadurch gekennzeichnet, dass** SEQ ID Nr. 1 und/oder 3 bzw. SEQ ID Nr. 2 und/oder 4 als ein Fusionsprotein bzw. als eine für ein Fusionsprotein kodierende Nukleinsäure vorliegt.

14. Verwendung mindestens eines FGF-BP Polypeptids gemäß SEQ ID Nr. 1 und 3 und/oder für FGF-BP Polypeptid kodierender Nukleinsäuren gemäß SEQ ID Nr. 2 und 4 und/oder gegen ein FGF-BP Polypeptid gemäß SEQ ID Nr. 1 oder SEQ ID Nr. 3 gerichteter Antikörper oder Antikörperfragmente und/oder mindestens einer FGF-BP gemäß SEQ ID Nr. 1 oder SEQ ID Nr. 3 exprimierenden Zelle zur Herstellung eines Diagnostikums zur Diagnose von diabetes-assoziierten Wundheilungsstörungen.

15. Verwendung nach Anspruch 14, **dadurch gekennzeichnet, dass** mindestens eine für FGF-BP Polypeptid kodierenden Nukleinsäure gemäß SEQ ID Nr. 2 und 4 als Sonde, vorzugsweise als DNA-Sonde und/oder als Primer zur Diagnose eingesetzt wird.

16. Verwendung nach Anspruch 14, **dadurch gekennzeichnet, dass** ein gegen ein FGF-BP Polypeptid gerichteter Antikörper oder Antikörperfragmente verwendet werden.

17. Verwendung nach mindestens einem der Ansprüche 1-16, **dadurch gekennzeichnet, dass** die diabetische Wundheilungsstörung oder die diabetes-assoziierte Wunde ein diabetischer Ulkus ist.

## Claims

1. Use of at least one FGF-BP polypeptide as depicted in SEQ ID No. 1 and SEQ ID No. 3, or of at least one FGF-BP-encoding nucleic acid as depicted in SEQ ID No. 2 and SEQ ID No. 4, or of antibodies or antibody fragments which are directed against an FGF-BP polypeptide as depicted in SEQ ID No. 1 or SEQ ID No. 3, and/or of a cell which is expressing an FGF-BP polypeptide as depicted in SEQ ID No. 1 or SEQ ID No. 3, for producing a drug for treating, diagnosing and/or preventing diabetes-associated wound healing disturbances.

2. Use according to Claim 1, **characterized in that** the drug comprises suitable additives and/or auxiliary substances.

3. Use according to Claim 1 or 2, **characterized in that** the nucleic acid as depicted in SEQ ID No. 2 and 4 is used in the form of an expression vector, in particular a vector which is active in gene therapy.

4. Use according to Claim 1 or 2, **characterized in that** the said cells are human, nonembryonic, autologous or allogenic cells.

5. Use according to Claim 4, **characterized in that** the cells are skin cells, in particular keratinocytes, fibroblasts or endothelial cells.

6. Use of at least one FGF-BP polypeptide as depicted in SEQ ID No. 1 and 3 and/or of at least one FGF-BP-encoding nucleic acid as depicted in SEQ ID No. 2 and 4, or of antibodies or antibody fragments which are directed against an FGF-BP polypeptide as depicted in SEQ ID No. 1 or SEQ ID No. 3 and/or of at least one cell which is expressing an FGF-BP polypeptide as depicted in SEQ ID No. 1 or SEQ ID No. 3, for identifying, in vitro, pharmacologically active substances which increase the function and/or expression of the FGF-BP polypeptide and/or of an FGF-BP polypeptide-encoding nucleic acid, for the purpose of treating and/or preventing diabetes-associated wounds.

7. Use of at least one FGF-BP polypeptide as depicted in SEQ ID No. 1 and 3, and/or of at least one FGF-BP-encoding nucleic acid as depicted in SEQ ID No. 2 and 4, or of antibodies or antibody fragments which are directed against an FGF-BP polypeptide as depicted in SEQ ID No. 1 or SEQ ID No. 3, and/or of at least one cell which is expressing an FGF-BP polypeptide as depicted in SEQ ID No. 1 or SEQ ID No. 3, for identifying, in vitro, pharmacologically active substances which increase the function and/or expression of the FGF-BP polypeptide and/or of an FGF-BP polypeptide-encoding nucleic acid, for the purpose of treating and/or preventing diabetic wound healing disturbances.

8. Use of at least one FGF-BP polypeptide as depicted in SEQ ID No. 1 and 3, and/or of at least one FGF-BP-encoding nucleic acid as depicted in SEQ ID No. 2 and 4, or of antibodies or antibody fragments which are directed against an FGF-BP polypeptide as depicted in SEQ ID No. 1 or SEQ ID No. 3, and/or of at least one cell which is expressing an FGF-BP polypeptide as depicted in SEQ ID No. 1 or SEQ ID No. 3, for producing a test for finding substances which are pharmacologically active in connection with diabetes-associated wounds.

9. Use according to Claim 8, **characterized in that** the test comprises suitable additives and/or auxiliary substances.

10. Use according to at least one of Claims 1, 6, 7, 8 and 9, **characterized in that** the nucleic acid is a DNA or RNA, preferably a DNA, particularly preferably a double-stranded DNA.

11. Use according to at least one of Claims 6, 7, 8 and 9, **characterized in that** at least one FGF-BP polypeptide, or nucleic acids encoding them, or antibodies or antibody fragments which are directed against FGF-BP polypeptides, are bound to a solid phase and the pharmacological activity of at least one substance is investigated.

12. Use according to at least one of Claims 6, 7, 8 and 9, **characterized in that** at least one FGF-BP polypeptide is expressed by at least one cell and the pharmacological activity of at least one substance is investigated.

13. Use according to at least one of Claims 1, 6, 7, 8 and 9, **characterized in that** SEQ ID No. 1 and/or 3 and, respectively, SEQ ID No. 2 and/or 4, is present as a fusion protein and, respectively, as a nucleic acid which encodes a fusion protein.

14. Use of at least one FGF-BP polypeptide as depicted in SEQ ID No. 1 and 3, and/or of FGF-BP polypeptide-encoding nucleic acids as depicted in SEQ ID No. 2 and 4, and/or of antibodies or antibody fragments which are directed against an FGF-BP polypeptide as depicted in SEQ ID No. 1 or SEQ ID No. 3, and/or of at least one cell which is expressing an FGF-BP as depicted in SEQ ID No. 1 or SEQ ID No. 3, for producing a diagnostic reagent for diagnosing diabetes-associated wound healing disturbances.

15. Use according to Claim 14, **characterized in that** use is made of at least one FGF-BP polypeptide-encoding nucleic acid as depicted in SEQ ID No. 2 and 4 as a probe, preferably as a DNA probe and/or as a primer for diagnosis.

16. Use according to Claim 14, **characterized in that** use is made of an antibody which is directed against an FGF-BP polypeptide or of antibody fragments.

17. Use according to at least one of Claims 1 - 16, **characterized in that** the diabetic wound healing disturbance or the diabetes-associated wound is a diabetic ulcer.

## Revendications

1. Utilisation d'au moins un polypeptide FGF-BP selon SEQ ID N° 1 et SEQ ID N° 3 ou d'au moins un acide nucléique codant pour le FGF-BP selon SEQ ID N° 2 et SEQ ID N° 4 ou d'anticorps ou de fragments d'anticorps dirigés contre un polypeptide FGF-BP selon SEQ ID N° 1 ou SEQ ID N° 3 et/ou d'une cellule exprimant un polypeptide FGF-BP selon SEQ ID N° 1 ou SEQ ID N° 3 pour la fabrication d'un médicament pour le traitement, le diagnostic et/ou la prévention de troubles de la cicatrisation associés au diabète.

2. Utilisation selon la revendication 1, **caractérisée en ce que** le médicament présente des additifs et/ou des agents auxiliaires appropriés.

3. Utilisation selon la revendication 1 ou 2, **caractérisée en ce que** l'acide nucléique selon SEQ ID N° 2 et 4 sont introduits sous la forme d'un vecteur d'expression, en particulier d'un vecteur efficace du point de vue génothérapeutique.

4. Utilisation selon la revendication 1 ou 2, **caractérisée en ce qu'**il s'agit pour lesdites cellules, de cellules humaines, non embryonnaires, autologues ou allogènes.

5. Utilisation selon la revendication 4, **caractérisée en ce que** les cellules sont des cellules cutanées, en particulier des kératinocytes, des fibroblastes ou des cellules endothéliales.

6. Utilisation d'au moins un polypeptide FGF-BP selon SEQ ID N° 1 et 3 et/ou d'au moins un acide nucléique codant pour le FGF-BP selon SEQ ID N° 2 et 4 ou d'anticorps ou de fragments d'anticorps dirigés contre un polypeptide FGF-BP selon SEQ ID N° 1 ou SEQ ID N° 3 et/ou d'au moins une cellule exprimant un polypeptide FGF-BP selon SEQ ID N° 1 ou SEQ ID N° 3 pour l'identification *in vitro* de substances pharmacologiquement actives, qui augmentent la fonction et/ou l'expression du polypeptide FGF-BP et/ou d'un acide nucléique codant pour le polypeptide FGF-BP pour le traitement et/ou la prévention de lésions associées au diabète.

7. Utilisation d'au moins un polypeptide FGF-BP selon SEQ ID N° 1 et 3 et/ou d'au moins un acide nucléique codant pour le FGF-BP selon SEQ ID N° 2 et 4 ou d'anticorps ou de fragments d'anticorps dirigés contre un polypeptide FGF-BP selon SEQ ID N° 1 ou SEQ ID N° 3 et/ou d'au moins une cellule exprimant un polypeptide FGF-BP selon SEQ ID N° 1 ou SEQ ID N° 3 pour l'identification *in vitro* de substances pharmacologiquement actives, qui augmentent la fonction et/ou l'expression du polypeptide FGF-BP et/ou d'un acide nucléique codant pour le polypeptide FGF-BP pour le traitement et/ou la prévention de troubles de la cicatrisation associés au diabète.

8. Utilisation d'au moins un polypeptide FGF-BP selon SEQ ID N° 1 et 3 et/ou d'au moins un acide nucléique codant pour le FGF-BP selon SEQ ID N° 2 et 4 ou d'anticorps ou de fragments d'anticorps dirigés contre un polypeptide FGF-BP selon SEQ ID N° 1 ou SEQ ID N° 3 et/ou d'au moins une cellule exprimant un polypeptide FGF-BP selon SEQ ID N° 1 ou SEQ ID N° 3 pour la fabrication d'un test de mise en évidence de substances pharmacologiquement actives en relation avec des lésions associées au diabète.

9. Utilisation selon la revendication 8, **caractérisée en ce que** le test comporte des additifs et/ou des agents auxiliaires appropriés.

10. Utilisation selon au moins l'une quelconque des revendications 1, 6, 7, 8 et 9, **caractérisée en ce que** l'acide nucléique est un ADN ou un ARN, de préférence un ADN, de manière particulièrement préférée, un ADN double brin.

11. Utilisation selon au moins l'une quelconque des revendications 6, 7, 8 et 9, **caractérisée en ce qu'**au moins un polypeptide FGF-BP ou des acides nucléiques codant pour ceux-ci ou des anticorps ou des fragments d'anticorps dirigés contre le polypeptide FGF-BP sont liés à une phase solide, et au moins une substance est étudiée pour son activité pharmacologique.

12. Utilisation selon au moins l'une quelconque des revendications 6, 7, 8 et 9, **caractérisée en ce qu'**au moins un polypeptide FGF-BP est exprimé à partir d'au moins une cellule et au moins une substance est étudiée pour son activité pharmacologique.

13. Utilisation selon au moins l'une quelconque des revendications 1, 6, 7, 8 et 9, **caractérisée en ce que** SEQ ID N° 1 et/ou 3 ou SEQ ID N° 2 et/ou 4 sont présents en tant que protéine de fusion ou en tant qu'acide nucléique codant pour une protéine de fusion.

14. Utilisation d'au moins un polypeptide FGF-BP selon SEQ ID N° 1 et 3 et/ou d'acides nucléiques codant pour le polypeptide FGF-BP selon SEQ ID n° 2 et 4 et/ou d'anticorps ou de fragments d'anticorps dirigés contre un polypeptide FGF-BP selon SEQ ID N° 1 ou SEQ ID N° 3 et/ou d'au moins une cellule exprimant le FGF-BP selon SEQ ID N° 1 ou SEQ ID N° 3 pour la fabrication d'un réactif de diagnostic pour le diagnostic de troubles de la cicatrisation associés au diabète.

15. Utilisation selon la revendication 14, **caractérisée en ce qu'**au moins un acide nucléique codant pour le polypeptide FGF-BP selon SEQ ID N° 2 et 4 est introduit en tant que sonde, de préférence en tant que sonde ADN et/ou en tant qu'amorce pour le diagnostic.

16. Utilisation selon la revendication 14, **caractérisée en ce qu'**un anticorps dirigé contre le polypeptide FGF-BP ou des fragments d'anticorps sont utilisés.

17. Utilisation selon au moins l'une quelconque des revendications 1 à 16, **caractérisée en ce que** le trouble de la cicatrisation associé au diabète ou la lésion associée au diabète est un ulcère diabétique.
